# EUROPEAN PATENT APPLICATION

(11) **EP 3 882 241 A1**
(43) Date of publication of application: **22.09.2021**
(21) Application number: 19885781.5
(22) Date of filing: 13.11.2019
(51) Int. Cl.: C07D 401/04, A61K 31/517, A61P 35/00

(54) **INHIBITOR OF BRUTON'S TYROSINE KINASE**

(30) Priority: 13.11.2018 CN 201811351513
(71) Applicant: Beijing Reciprocapharmaceuticals Co., Ltd., Beijing 102206 (CN)
(72) Inventor: PAN, Zhengying, Beijing 102206 (CN); JIAO, Zhaodong, Beijing 102206 (CN); LI, Xitao, Beijing 102206 (CN); TAO, Guanyu, Beijing 102206 (CN); SHI, Yanxia, Beijing 102206 (CN); ZHANG, Rui, Beijing 102206 (CN)
(74) Representative: Plasseraud IP
(86) International application number: PCT/CN2019/118136
(87) International publication number: WO 2020/098716

(57) **Abstract**

Disclosed herein is a compound of Formula (I) with a Btk inhibitory activity, wherein all the variables are as defined herein. The compound can be used for the treatment of diseases such as autoimmune diseases, xenogeneic immune diseases, cancers or thromboembolic diseases. Also disclosed is a pharmaceutical composition comprising a compound of Formula (I). Futher provided is a compound capable of inhibiting the activity of Bruton's tyrosine kinase by covalent binding.

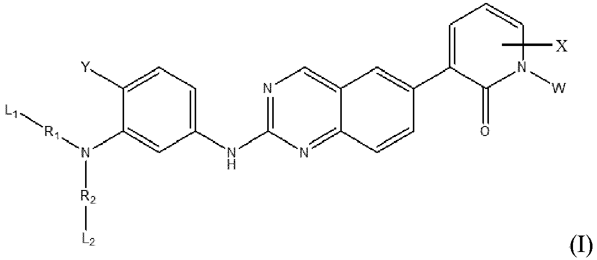

## Description

### TECHNICAL FIELD

The present invention relates to a compound that inhibits the activity of Bruton's tyrosine kinase (Btk). The compound of the present invention has a novel skeleton structure and can be used to treat diseases related to B cells.

### BACKGROUND OF THE INVENTION

"Molecular targeted therapy" is a method for treating cancer with some key enzymes involved in the differentiation and proliferation of tumor cells as targets for drug screening, which has become an important field in research and development of anti-cancer drugs. In recent years, protein tyrosine kinase (PTK) signaling pathway has become a focus of researches, and much has been learned about the relationship between PTKs and proliferation, differentiation, migration, and apoptosis of tumor cells. Strategies for interfering with or blocking the tyrosine kinase pathway have been used in the treatment of tumors and thus development of PTK inhibitors with high inhibitory activity becomes an important method for developing novel anti-tumor drugs.

In PTKs, Bruton's tyrosine kinase (Btk) belongs to the Tec family of non-receptor tyrosine kinases. Btk plays a vital role in the B cell signaling pathway that connects the stimulation of the B-cell receptor (BCR) on the cell surface to the downstream intracellular response, and is a critical modulator for development, activation, signaling and survival of B cells. Therefore, many human diseases associated with B cells are related to overexpression of Btk. Studies have proved that the transmission of tumor cells can be destroyed by blocking the operation of tyrosine kinases, thereby achieving the purpose of inhibiting tumors. As a result, a Btk inhibitor represents a treatment means for effectively preventing B cell mediation, and is mainly used clinically to treat X-linked agammaglobulinemia (XLA), chronic lymphocytic leukemia (CLL), diffuse giant B cell lymphoma, etc. In addition, studies show that Btk is involved in a variety of life signal processes and regulation of tumor tissue microenvironment (see Literature 5), and is also a therapeutic target for autoimmune diseases, xenogeneic immune diseases, inflammatory diseases, thromboembolic diseases, and solid tumors.

The most successful Btk inhibitor is ibrutinib, which was co-developed by one of the inventors of the present application (see Literatures 1 and 2). The drug was designated by the FDA as a "breakthrough" new drug, which has a broad prospect for research and development. However, there is still a need for development of more Btk inhibitors.

Some of the inventors of the present application reported a (aminophenylamino)pyrimidinylbenzamide inhibitor in previous patent applications (see Literature 3), which was confirmed to have a Btk inhibitory activity.

Additionally, current protein tyrosine kinase inhibitors with a quinazoline structure include drugs such as gefitinib, erlotinib, lapatinib, etc. However, they mainly target epidermal growth factor receptor (EGFR) tyrosine kinase, and their skeleton structures are basically 4-anilinoquinazoline. In addition, Literature 4 discloses a compound with aminoquinazoline as the backbone, but it targets Lck kinase of the Src family.

In view of the above background, there is still a need for development of more high-efficiency inhibitors against Btk, and priority should be given to development of compounds that inhibit the activity of Bruton's tyrosine kinase in a manner of covalent binding.

### Prior art literatures

Literature 1: CN101610676A
Literature 2: Zhengying Pan, et al., Chem Med Chem, 2007, 2, pp. 58-61
Literature 3: WO2013060098A1
Literature 4: Erin F. Dimauro, et al., J. Med. Chem., 2006, 49, pp. 5671-5686
Literature 5: Pal Singh S, et al., Mol Cancer, 19 Feb 2018;17(1):57

### SUMMARY OF THE INVENTION

The problem to be solved by the invention

The objective of the present invention is to provide a high-efficiency kinase inhibitor compound against Btk with a novel skeleton structure.

### Means to solve the problem

In one aspect of the present invention, there is provided a compound of Formula (I): 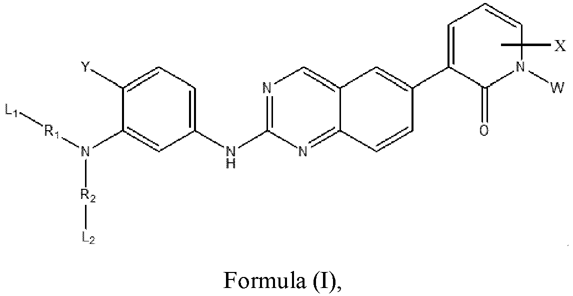 wherein,
W is selected from the group consisting of H, C₁₋₆alkyl, aryl optionally substituted with halogenated C₁₋₃alkyl or a five to seven-membered nitrogen-containing aliphatic ring, heteroaryl, and -C(O)-R₃, wherein R₃ is C₁₋₃alkyl, di(C₁₋₃alkyl)amino, or C₁₋₃alkoxy optionally substituted with aryl, the aryl includes phenyl, naphthalenyl, phenanthrenyl, anthracenyl, fluorenyl, indenyl, or the like, the heteroaryl includes: 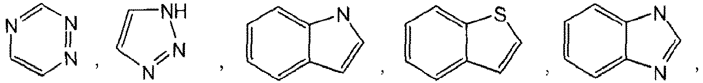 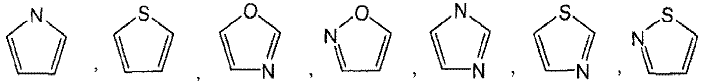 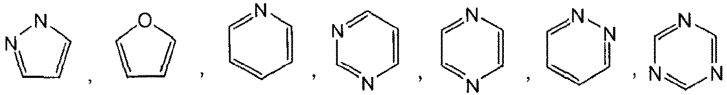 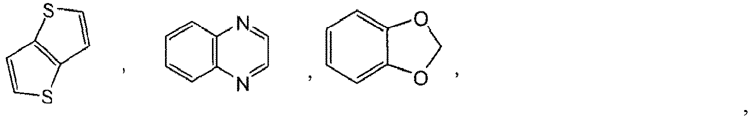 or the like;
X is selected from the group consisting of halo and C₁₋₆alkyl, and X is bonded to any bondable position on the pyridone ring;
Y is selected from the group consisting of H, halo, C₁₋₆alkyl, and C₁₋₃alkoxy optionally substituted with C₁₋₃alkoxy;
R₁ and R₂ are independently the same or different, and are selected from the group consisting of H, C₁₋₃alkyl optionally substituted with C₁₋₃alkoxy or di(C₁₋₃alkyl)amino, C(O), S(O) and S(O)₂;
L₁ and L₂ are independently the same or different, and are selected from the group consisting of C₁₋₆alkyl optionally substituted with -NR₄R₅, C₂₋₃alkenyl optionally substituted with halo or optionally substituted C₁₋₃alkyl, and C₂₋₃alkynyl optionally substituted with C₁₋₃alkyl, wherein the substituent of the optionally substituted C₁₋₃alkyl is di(C₁₋₃alkyl)amino, or a five to seven-membered nitrogen-containing aliphatic ring, and
R₄ and R₅ in NR₄R₅ are independently the same or different, and are selected from the group consisting of H, C₁₋₃alkyl, and C₂₋₃alkenylcarbonyl, or one of R₄ and R₅, and one carbon atom in the C₁₋₆alkyl optionally substituted with -NR₄R₅, together with the atom to which they are bonded, form a five- to seven-membered nitrogen-containing aliphatic ring,
provided that when R₁ is H, or C₁₋₃alkyl optionally substituted with C₁₋₃alkoxy or di(C₁₋₃alkyl)amino, L₁ is absent, and when R₂ is H, or C₁₋₃alkyl optionally substituted with C₁₋₃alkoxy or di(C₁₋₃alkyl)amino, L₂ is absent,
or a pharmaceutically acceptable salt thereof

In a preferred embodiment, the -N(R₁-L₁)(R₂-L₂) in Formula (I) comprises at least one unsaturated carbon-carbon bond. In a further preferred embodiment, the compound of Formula (I) comprising at least one unsaturated carbon-carbon bond in the -N(R₁-L₁)(R₂-L₂) inhibits the activity of Bruton's tyrosine kinase in a manner of irreversible covalent binding (for example, by forming a covalent bond with an amino acid residue on Bruton's tyrosine kinase). In a preferred aspect, the unsaturated carbon-carbon bond is a carbon-carbon double bond or a carbon-carbon triple bond.

In a preferred embodiment,
W is selected from the group consisting of H, aryl optionally substituted with -CF₃ or 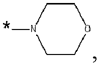 and -C(O)-R₃, wherein R₃ is C₁₋₃alkoxy optionally substituted with aryl;
Y is selected from the group consisting of H, halo, and C₁₋₃alkoxy;
R₁ and R₂ are independently the same or different, and are selected from the group consisting of H, C₁₋₃alkyl optionally substituted with C₁₋₃alkoxy or di(C₁₋₃alkyl)amino, and C(O);
L₁ and L₂ are independently the same or different, and are selected from the group consisting of C₁₋₆alkyl substituted with -NR₄R₅, C₂₋₃alkenyl optionally substituted with substituted C₁₋₃alkyl, and C₂₋₃alkynyl optionally substituted with C₁₋₃alkyl, wherein the substituent of the substituted C₁₋₃alkyl is dimethylamino, 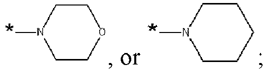 and
R₄ and R₅ in -NR₄R₅ are independently the same or different, and are selected from the group consisting of H, C₁₋₃alkyl, and C₂₋₃alkenylcarbonyl, or one of R₄ and R₅, and one carbon atom in the C₁₋₆alkyl substituted with -NR₄R₅, together with the atom to which they are bonded, form a tetrahydropyrrole ring.

In another preferred embodiment,
W is selected from H;
X is selected from C₁₋₆alkyl;
Y is selected from the group consisting of H and C₁₋₃alkoxy;
R₁ and R₂ are independently the same or different, and are selected from the group consisting of H, C₁₋₃alkyl optionally substituted with C₁₋₃alkoxy, and C(O);
L₁ and L₂ are independently the same or different, and are selected from the group consisting of C₁₋₆alkyl substituted with -NR₄R₅, C₂₋₃alkenyl optionally substituted with di(C₁₋₃alkyl)amino C₁₋₃alkyl, and C₂₋₃alkynyl optionally substituted with C₁₋₃alkyl; and
R₄ and R₅ in -NR₄R₅ are independently the same or different, and are selected from the group consisting of H and C₂₋₃alkenylcarbonyl.

In another preferred embodiment, one of R₁ and R₂ is H or C₁₋₃alkyl optionally substituted with C₁₋₃alkoxy or di(C₁₋₃alkyl)amino, and the other is C(O).

In another preferred embodiment, one of R₁ and R₂ is H, and the other is C₁₋₃alkyl optionally substituted with C₁₋₃alkoxy or di(C₁₋₃alkyl)amino.

In another preferred embodiment, the -N(R₁-L₁)(R₂-L₂) is selected from the group consisting of - NR₆-C(O)-CH=CH₂, -NR₆-C(O)-C≡C-CH₃, -NR₆-C(O)-CH=CH₂CH₂N(CH₂)₂, 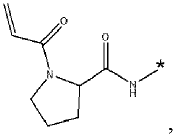 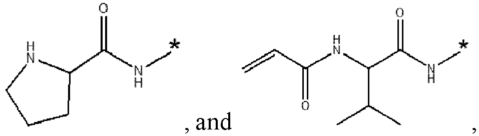 wherein, R₆ is H, or C₁₋₃alkyl optionally substituted with C₁₋₃alkoxy.

In another preferred embodiment the -N(R₁-L₁)(R₂-L₂₎ is selected from the group consisting

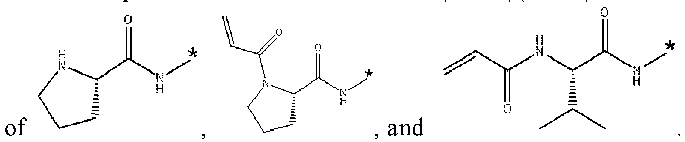

In another preferred embodiment, W is selected from H, X is selected from methyl, Y is selected from the group consisting of H and methoxy, and the -N(R₁-L₁)(R₂-L₂) is -NR₆-C(O)-CH=CH₂, wherein R₆ is H, or C₁₋₃alkyl optionally substituted with C₁₋₃alkoxy.

In another preferred embodiment, when Y is C₁₋₆alkyl, or C₁₋₃alkoxy optionally substituted with C₁₋₃alkoxy, and one of R₁ and R₂ is C₁₋₃alkyl optionally substituted with C₁₋₃alkoxy or di(C₁₋₃alkyl)amino, one of R₁ and R₂, and Y, together with the atom to which they are bonded, can form a five- to seven-membered nitrogen-containing aliphatic ring.

In another preferred embodiment, when Y is C₁₋₃alkoxy optionally substituted with C₁₋₃alkoxy, and one of R₁ and R₂ is C₁₋₃alkyl optionally substituted with C₁₋₃alkoxy or di(C₁₋₃alkyl)amino, one of R₁ and R₂, and Y, together with the atom to which they are bonded, can form a morpholine ring.

In another aspect of the present invention, the following compounds or pharmaceutically acceptable salts thereof are provided, wherein the compound is selected from the group consisting of: 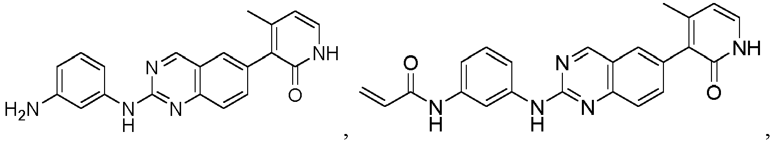 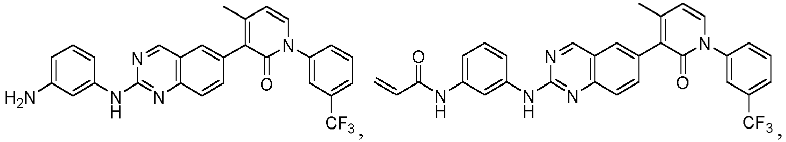 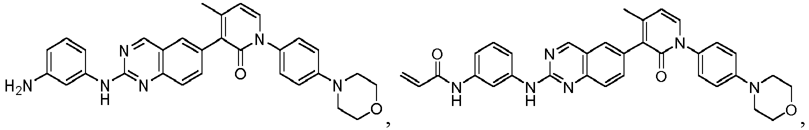 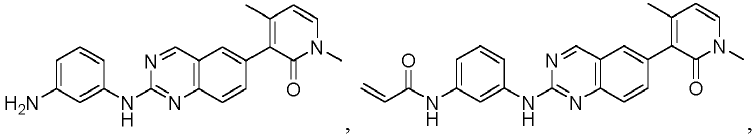 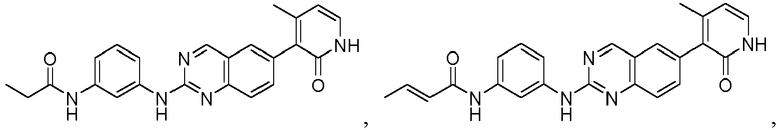 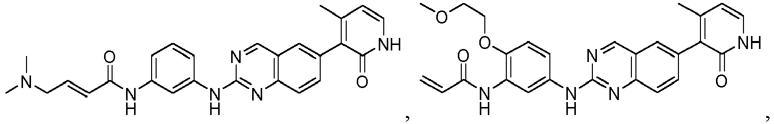 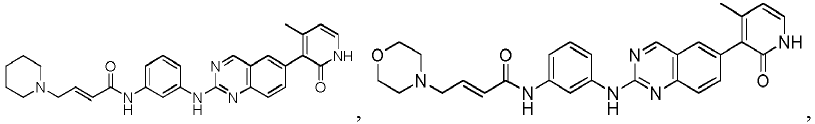 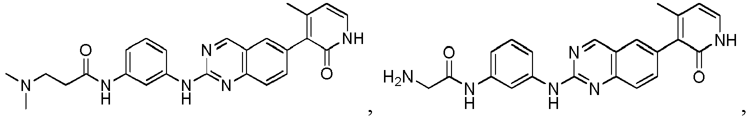 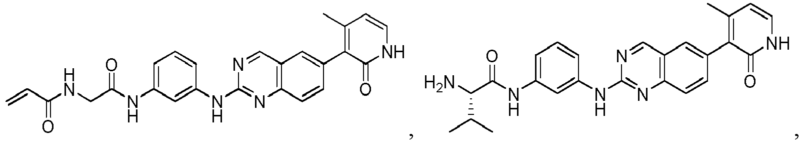 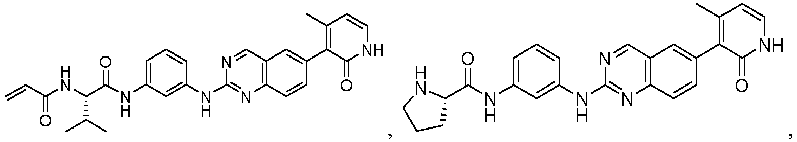 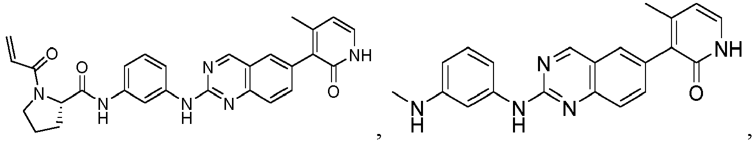 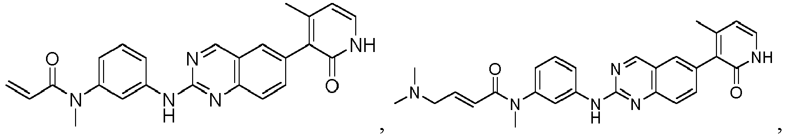 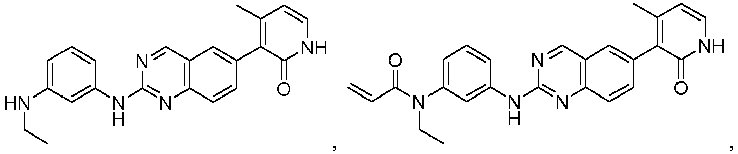 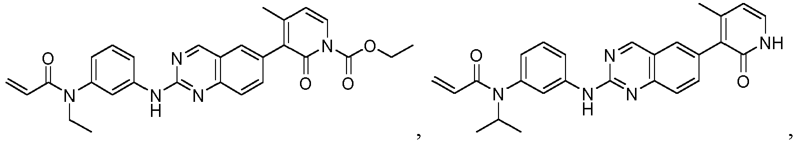 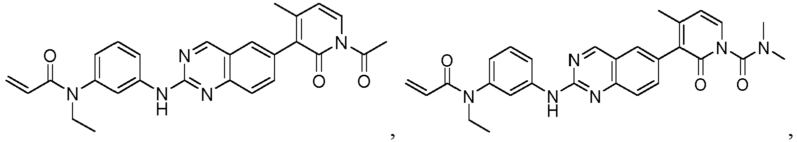 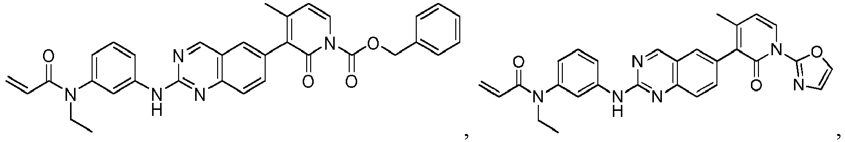 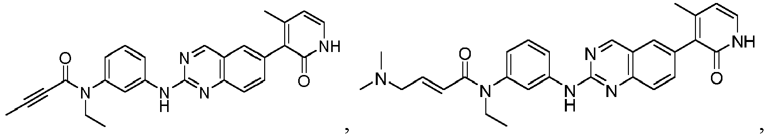 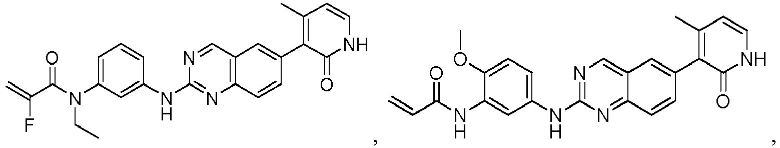 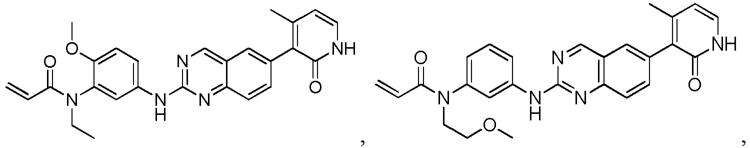 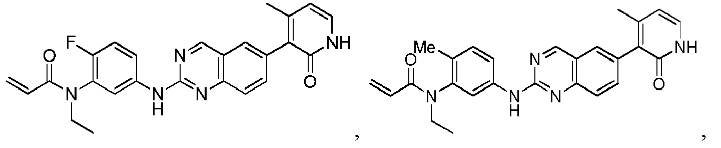 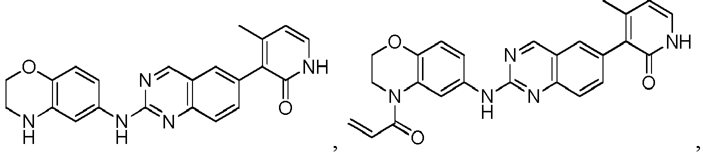 and

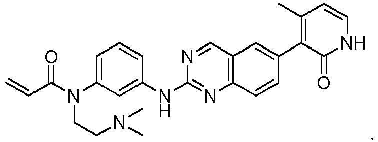

The compound is further selected from the group consisting of:

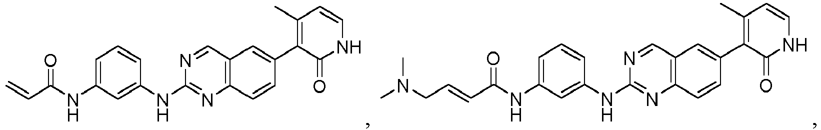

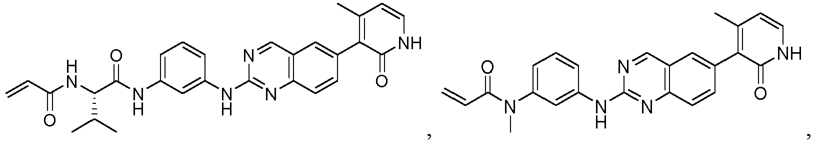

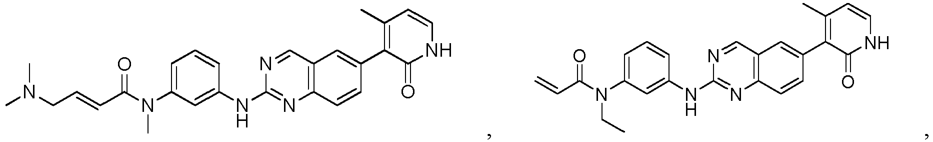

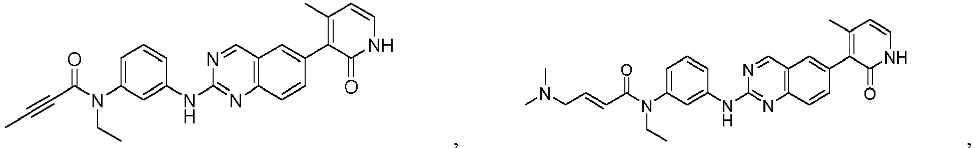

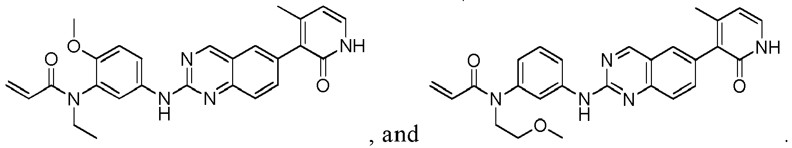

The compound of the present invention can be prepared or used as a pharmaceutically acceptable salt thereof. This can be done by any salt-forming means known in the art. For example, the pharmaceutically acceptable salt can be an acid addition salt, such as an inorganic acid addition salt or an organic acid addition salt. For example, the pharmaceutically acceptable salt can also be a salt formed by replacing an acidic proton in the compound with a metal ion, or a salt formed by coordination of the compound with an organic base or an inorganic base.

The present invention provides an inhibitor of Bruton's tyrosine kinase. Further, the present invention provides an inhibitor compound that inhibits the activity of Bruton's tyrosine kinase in a manner of covalent binding. In particular, the present invention provides a compound capable of forming a covalent bond with an amino acid residue on Bruton's tyrosine kinase.

In another aspect of the present invention, there is provided a pharmaceutical composition comprising an effective amount of the above compound of the present invention or a pharmaceutically acceptable salt thereof, and a pharmaceutically acceptable carrier or excipient.

The present invention also provides use of the compound described herein or a pharmaceutically acceptable salt thereof in the manufacture of a medicament for inhibiting the activity of Bruton's tyrosine kinase. In a preferred aspect, the inhibiting is performed by covalent binding. In a further preferred aspect, the inhibiting is achieved by forming a covalent bond between the compound and an amino acid residue on Bruton's tyrosine kinase.

In another aspect of the present invention, there is provided use of the compound of the present invention or a pharmaceutically acceptable salt thereof in the manufacture of a medicament for treating the following diseases or conditions: autoimmune diseases, xenogeneic immune diseases, inflammatory diseases, cancers, and thromboembolic diseases.

The effect of the invention

The present invention provides a high-efficiency kinase inhibitor compound against Btk with (aminophenylamino)quinazoline linked to a 2-pyridone ring as the backbone.

### BRIEF DESCRIPTION OF THE DRAWINGS

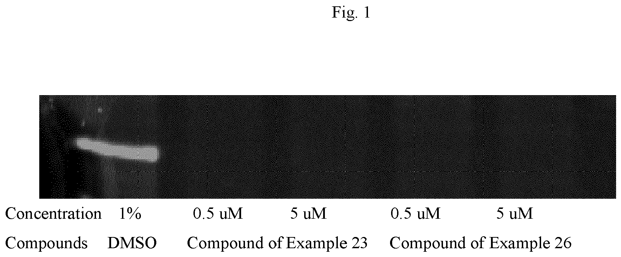

Fig. 1 is a graph showing the scanning results from SDS-PAGE electrophoresis after the compounds of Examples 23 and 26 at two concentrations (0.5 µM and 5 µM) were incubated with BTK for 1 h, and then a probe prepared according to Sci. Rep. 2015, 5, 16136 was added and incubated for 1 h. The results show the inhibitory effect of the compound at two concentrations on the fluorescent probe-labeled BTK.

### DETAILED DESCRIPTION OF THE INVENTION

Unless defined otherwise, all technical and scientific terms used herein have the same meaning as is commonly understood by one of skill in the art to which the claimed subject matter belongs. Definition of standard chemistry terms can be found in reference works, including Carey and Sundberg, Advanced Organic Chemistry, 4th Ed., Vols. A (2000) and B (2001), Plenum Press, New York.

"C₁₋₆alkyl" refers to an alkyl group with 1-6 carbon atoms, including methyl, ethyl, propyl, butyl, pentyl and hexyl, including all possible isomeric forms, such as n-propyl and isopropyl, n-butyl, isobutyl, sec-butyl, tert-butyl, etc. "C₁₋₆alkyl" includes all sub-ranges contained therein, such as C₁₋₂alkyl, C₁₋₃alkyl, C₁₋₄alkyl, C₁₋₅alkyl, C₂₋₅alkyl, C₃₋₅alkyl, C₄₋₅alkyl, C₃₋₄alkyl, C₃₋₅alkyl and C₄₋₅alkyl. "C₁₋₃alkyl" includes methyl, ethyl, n-propyl and isopropyl. "C₂₋₃alkenyl" includes vinyl (-CH=CH₂), propenyl (-CH=CHCH₃), and isopropenyl (-C(CH₃)=CH₂). "C₂₋₃alkynyl" includes ethynyl (-C=CH) and propynyl (-C=CCH3). An aromatic group refers to a planar ring having a delocalized *π*-electron system containing 4n+2 *π* electrons, where n is an integer. Aromatic groups can be formed from five, six, seven, eight, nine, or more than nine ring atoms. Aromatic groups can be optionally substituted. Aromatic groups include "aryl" (in which ring atoms only consist of carbon atoms) and "heteroaryl" (in which ring atoms consist of carbon atoms and one or more heteroatoms selected from the group consisting of, for example, oxygen, sulfur, and nitrogen). "Aryl" and "heteroaryl" include monocyclic or fused polycyclic (i.e., rings which share adjacent pairs of ring atoms) groups. Examples of "aryl" include, but are not limited to phenyl, naphthalenyl, phenanthrenyl, anthracenyl, fluorenyl, and indenyl.

Examples of "heteroaryl" include: 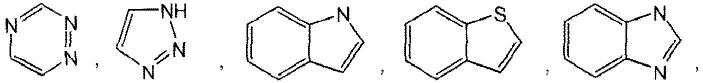 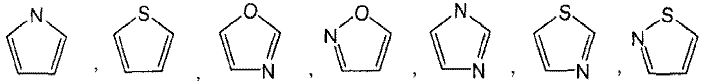 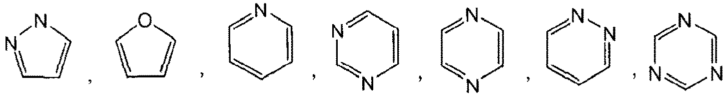 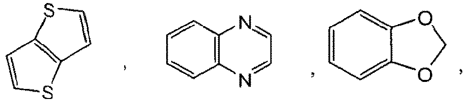 and the like.

"Halo" refers to fluoro, chloro, bromo and iodo. "C₁₋₃alkoxy" refers to (C₁₋₃alkyl)O-, where C₁₋₃alkyl is as defined herein. Di(C₁₋₃alkyl)amino refers to di(C₁₋₃alkyl)N-, where C₁₋₃alkyl is as defined herein. C₂₋₃alkenylcarbonyl refers to (C₂₋₃alkenyl)C(O)-, where C₂₋₃alkenyl is as defined herein. "Halogenated C₁₋₃alkyl" refers to halo-(C₁₋₃alkyl)-, where C₁₋₃alkyl is as defined herein. halogenated C₁₋₃alkyl includes perhalogenated C₁₋₃alkyl, in which all hydrogen atoms in the C₁₋₃alkyl are replaced with halogen, such as -CF₃, -CH₂CF₃, -CF₂CF₃, -CH₂CH₂CF₃, or the like.

The five- to seven-membered nitrogen-containing aliphatic ring refers to a saturated monocyclic ring containing at least one nitrogen atom, in which each ring comprises 5, 6 or 7 atoms (except for hydrogen atoms or a substituent when the hydrogen atom is substituted). Examples thereof include morpholinyl, piperazinyl, piperidinyl, azepanyl, aziridinyl, diazepanyl, 1,3-diazacyclohexane, 1,4-diazacyclohexane, pyrrolidinyl, oxazolidinyl, thiazolidinyl, isoxazolidinyl, thiomorpholinyl, and imidazolinyl. For example, a heterocyclic group such as a five- to seven-membered nitrogen-containing aliphatic ring is connected to the main structure of the compound through any substitutable carbon atom or any substitutable nitrogen atom contained in the group, preferably through a nitrogen atom.

The term "pharmaceutically acceptable", with respect to a formulation, composition or ingredient, as used herein, means having no persistent detrimental effect on the general health of the subject being treated or does not abrogate the biological activity or properties of the compound, and is relatively nontoxic.

The term "Bruton's tyrosine kinase" as used herein refers to Bruton's tyrosine kinase *from Homo sapiens,* as disclosed in, e.g., US Patent No. 6326469 (GenBank Accession No. NP 000052).

The terms "effective amount" or "therapeutically effective amount", as used herein, refer to a sufficient amount of an agent or a compound being administered which will relieve to some extent one or more of the symptoms of the disease or condition being treated. The result can be reduction and/or alleviation of the signs, symptoms, or causes of a disease, or any other desired alteration of a biological system. For example, an "effective amount" for therapeutic uses is an amount required to provide a clinically significant decrease in disease symptoms without undue adverse side effects. An appropriate "effective" amount in any individual case can be determined using techniques, such as a dose escalation study. The term "therapeutically effective amount" includes, for example, a prophylactically effective amount. An "effective amount" of a compound disclosed herein is an amount effective to achieve a desired pharmacologic effect or therapeutic improvement without undue adverse side effects. It is understood that "an effect amount" or "a therapeutically effective amount" can vary from subject to subject, due to variation in metabolism of the compound, age, weight, general condition of the subject, the condition being treated, the severity of the condition being treated, and the judgment of the prescribing physician. By way of example only, therapeutically effective amounts may be determined by routine experimentation, including but not limited to a dose escalation clinical trial.

The terms "inhibits", "inhibiting", or "inhibitor" of a kinase, as used herein, refer to inhibition of the activity of Bruton's tyrosine kinase.

The autoimmune diseases described herein include, but are not limited to, rheumatoid arthritis, psoriatic arthritis, osteoarthritis, Still's disease, juvenile arthritis, lupus, diabetes, myasthenia gravis, Hashimoto's thyroiditis, Ord's thyroiditis, Graves' disease, rheumatoid arthritis syndrome, multiple sclerosis, infective neuronitis, acute disseminated encephalomyelitis, Addison's disease, opsoclonusmyoclonus syndrome, ankylosing spondylitisis, antiphospholipid antibody syndrome, aplastic anemia, autoimmune hepatitis, coeliac disease, Goodpasture's syndrome, idiopathic thrombocytopenic purpura, optic neuritis, scleroderma, primary biliary cirrhosis, Reiter's syndrome, Takayasu's arteritis, temporal arteritis, warm autoimmune hemolytic anemia, Wegener's granulomatosis, psoriasis, alopecia universalis, Behçet's disease, chronic fatigue, dysautonomia, endometriosis, interstitial cystitis, neuromyotonia, scleroderma, and vulvodynia.

The xenogeneic immune diseases described herein include, but are not limited to, graft versus host disease, transplantation, transfusion, anaphylaxis, allergies (e.g., allergies to plant pollens, latex, drugs, foods, insect poisons, animal hair, animal dander, dust mites, or cockroach calyx), type I hypersensitivity, allergic conjunctivitis, allergic rhinitis, and atopic dermatitis.

The inflammatory diseases described herein include, but are not limited to, asthma, inflammatory bowel disease, appendicitis, blepharitis, bronchiolitis, bronchitis, bursitis, cervicitis, cholangitis, cholecystitis, colitis, conjunctivitis, cystitis, dacryoadenitis, dermatitis, dermatomyositis, encephalitis, endocarditis, endometritis, enteritis, enterocolitis, epicondylitis, epididymitis, fasciitis, fibrositis, gastritis, gastroenteritis, hepatitis, hidradenitis suppurativa, laryngitis, mastitis, meningitis, myelitis myocarditis, myositis, nephritis, oophoritis, orchitis, osteitis, otitis, pancreatitis, parotitis, pericarditis, peritonitis, pharyngitis, pleuritis, phlebitis, pneumonitis, pneumonia, proctitis, prostatitis, pyelonephritis, rhinitis, salpingitis, sinusitis, stomatitis, synovitis, tendonitis, tonsillitis, uveitis, vaginitis, vasculitis, and vulvitis.

The cancers, e.g., B-cell proliferative diseases, as described herein include, but are not limited to, diffuse large B cell lymphoma, follicular lymphoma, chronic lymphocytic lymphoma, chronic lymphocytic leukemia, B cell prolymphocytic leukemia, lymphoplasmacytic lymphoma/Waldenström macroglobulinemia, splenic marginal zone lymphoma, plasma cell myeloma, plasmacytoma, extranodal marginal zone B cell lymphoma, nodal marginal zone B cell lymphoma, mantle cell lymphoma, mediastinal (thymic) large B cell lymphoma, intravascular large B cell lymphoma, primary effusion lymphoma, burkitt lymphoma/leukemia, and lymphomatoid granulomatosis.

The thromboembolic diseases described herein include, but are not limited to, myocardial infarct, angina pectoris (including unstable angina), reocclusions or restenoses after angioplasty or aortocoronary bypass, stroke, transitory ischemia, peripheral arterial occlusive disorders, pulmonary embolisms, and deep venous thromboses.

Diagnosis of each symptom of the above diseases and evaluation of prognosis are known in the art. See, e.g., Harrison's Principles of Internal, 16th Ed., 2004, The McGraw-Hill Companies, Inc. Dey, et al. (2006), Cytojournal 3(24), and the "Revised European American Lymphoma" (REAL) classification system (see, e.g., the website maintained by the National Cancer Institute).

A number of animal models are useful for establishing a range of therapeutically effective doses of Btk inhibitor compounds for treating any of the foregoing diseases.

The therapeutic efficacy of the compound for one of the foregoing diseases can be optimized during a course of treatment. For example, a subject being treated can undergo a diagnostic evaluation to correlate the relief of disease symptoms or pathologies to inhibition of *in vivo* Btk activity achieved by administering a given dose of a Btk inhibitor. Cellular assays known in the art can be used to determine *in vivo* activity of Btk in the presence or absence of a Btk inhibitor. For example, since activated Btk is phosphorylated at tyrosine 223 (Y223) and tyrosine 551 (Y551), phospho-specific immunocytochemical staining of P-Y223 or PY551-positive cells can be used to detect or quantify activation of Bkt in a population of cells (e.g., by FACS analysis of stained vs. unstained cells). See, e.g., Nisitani, et al. (1999), Proc. Natl. Acad. Sci, USA 96:2221-2226. Thus, the amount of the Btk inhibitor compound that is administered to a subject can be increased or decreased as needed so as to maintain a level of Btk inhibition optimal for treating the subject's disease state.

The starting material used for the synthesis of the compounds described herein may be synthesized or can be obtained from commercial sources, such as, but not limited to, Aldrich Chemical Co.

(Milwaukee, Wisconsin), Bachem (Torrance, California), or Sigma Chemical Co. (St. Louis, Mo.). The compounds described herein, and other related compounds having different substituents can be synthesized using techniques and materials known to those of skill in the art, such as described, for example, in March, ADVANCED ORGANIC CHEMISTRY, 4th Ed., (Wiley 1992); Carey and Sundberg, ADVANCED ORGANIC CHEMISTRY, 4th Ed., Vols. A and B (Plenum 2000, 2001); Green and Wuts, PROTECTIVE GROUPS IN ORGANIC SYNTHESIS, 3rd Ed., (Wiley 1999); Fieser's Reagents for Organic Synthesis, Volumes 1-17 (John Wiley and Sons, 1991); Rodd's Chemistry of Carbon Compounds, Volumes 1-5 and Supplementals (Elsevier Science Publishers, 1989); Organic Reactions, Volumes 1-40 (John Wiley and Sons, 1991); and Larock's Comprehensive Organic Transformations (VCH Publishers Inc., 1989) (incorporated herein by reference in their entirety). Other methods for the synthesis of compounds described herein may be found in International Patent Publication No. WO 01/01982901, Arnold, et al., Bioorganic & Medicinal Chemistry Letters 10 (2000) 2167-2170; Burchat, et al., Bioorganic & Medicinal Chemistry Letters 12 (2002) 1687-1690. General methods for the preparation of compound as disclosed herein may be derived from reactions known in the art and the reactions may be modified by the use of appropriate reagents and conditions deemed appropriate by those skilled in the art, for the introduction of the various moieties found in the molecules as provided herein. The following synthetic method can be used as a guide.

The products of the reactions may be isolated and purified, if desired, using conventional techniques, including, but not limited to, filtration, distillation, crystallization, chromatography and the like. Such products may be characterized using conventional means, including physical constants and spectral data.

Compounds described herein can be prepared using the synthetic methods described herein as a single isomer or a mixture of isomers.

The compounds described herein can possess one or more stereocenters and each center can exist in the R or S configuration. The compounds presented herein include all diastereomeric, enantiomeric, and epimeric forms as well as the appropriate mixtures thereof. Stereoisomers may be obtained, if desired, by methods known in the art, for example, the separation of stereoisomers by chiral chromatographic columns.

Diasteromeric mixtures can be separated into their individual diastereomers on the basis of their physical chemical differences by methods known, for example, by chromatography and/or fractional crystallization. In one embodiment, enantiomers can be separated by chiral chromatographic columns. In other embodiments, enantiomers can be separated by converting the enantiomeric mixture into a diastereomeric mixture by reaction with an appropriate optically active compound (e.g., alcohol), separating the diastereomers and converting (e.g., hydrolyzing) the individual diastereomers to the corresponding pure enantiomers. All such isomers, including diastereomers, enantiomers, and mixtures thereof are considered as part of the compositions described herein.

The methods and formulations described herein include the use of N-oxides, crystalline forms (also known as polymorphs), or pharmaceutically acceptable salts of compounds described herein, as well as active metabolites of these compounds having the same type of activity. In some situations, compounds may exist as tautomers. All tautomers are included within the scope of the compounds presented herein. In addition, the compounds described herein can exist in unsolvated as well as solvated forms with pharmaceutically acceptable solvents such as water, ethanol, and the like. The solvated forms of the compounds presented herein are also considered to be disclosed herein.

An unoxidized form can be prepared from N-oxides by treating with a reducing agent, such as, but not limited to, sulfur, sulfur dioxide, triphenyl phosphine, lithium borohydride, sodium borohydride, phosphorus trichloride, tribromide, or the like in a suitable inert organic solvent, such as, but not limited to, acetonitrile, ethanol, aqueous dioxane, or the like at 0 to 80°C.

In some embodiments, compounds described herein are prepared as prodrugs. A "prodrug" refers to an agent that is converted into the parent drug *in vivo.* Prodrugs are often useful because, in some situations, they are easier to administer than the parent drug. Prodrugs may, for instance, be bioavailable by oral administration whereas the parent is not. The prodrug may have improved solubility in pharmaceutical compositions over the parent drug. Prodrugs may be designed as reversible drug derivatives, to enhance drug transport to site-specific tissues. In some embodiments, the design of a prodrug increases the effective water solubility. See, e.g., Fedorak, et al., Am. J. Physiol, 269: G210-218(1995); McLoed, et al., Gastroenterol, 106: 405-413(1994); Hochhaus, et al., Biomed. Chrom., 6:283-286 (1992); J. Larsen and H. Bundgaard, Int. J. Pharmaceutics, 37, 87(1987); J. Larsen, et al., Int. J. Pharmaceutics, 47, 103 (1988); Sinkula, et al., J. Pharm. Sd., 64:181-210 (1975); T. Higuchi and V. Stella, Pro-drugs as Novel Delivery Systems, the A.C.S. Symposium Series, Vol. 14; and Edward B. Roche, Carriers in Drug Design, American Pharmaceutical Association and Pergamon Press,1987, all incorporated herein by reference in their entirety. An example, without limitation, of a prodrug would be a compound as described herein which is administered as an ester (the "prodrug") to facilitate transmittal across a cell membrane where water solubility is detrimental to mobility but which then is metabolically hydrolyzed to the carboxylic acid, the active entity, once inside the cell where water solubility is beneficial. A further example of a prodrug might be a short peptide (polyamino acid) bonded to an acid group where the peptide is metabolized to reveal the active moiety. In certain embodiments, upon *in vivo* administration, a prodrug is chemically converted to the biologically, pharmaceutically or therapeutically active form of the compound. In certain embodiments, a prodrug is enzymatically metabolized by one or more steps or processes to the biologically, pharmaceutically or therapeutically active form of the compound. A pharmaceutically active compound can be modified to produce a prodrug such that the active compound will be regenerated upon *in vivo* administration. The prodrug can be designed to alter the metabolic stability or the transport characteristics of a drug, to mask side effects or toxicity, to improve the flavor of a drug or to alter other characteristics or properties of a drug. Based on knowledge of pharmacodynamic processes and drug metabolism *in vivo,* once a pharmaceutically active compound is known, those skilled in the art can design prodrugs of the compound (see, for example, Nogrady (1985) Medicinal Chemistry A Biochemical Approach, Oxford University Press, New York, pages 388-392; Silverman (1992), The Organic Chemistry of Drug Design and Drug Action, Academic Press, Inc., San Diego, pages 352-401, Saulnier, et al., (1994), Bioorganic and Medicinal Chemistry Letters, Vol. 4, p. 1985).

Prodrug forms of the herein described compounds, wherein the prodrug is metabolized *in vivo* to produce a derivative as set forth herein are included within the scope of the claims. In some cases, some of the herein-described compounds may be a prodrug or another derivative of active compound.

The compounds described herein encompass compounds comprising isotopes, which are identical to those recited herein in molecular formula and structural formula, but for the fact that one or more atoms are replaced by a nuclide that has the same element but has an atomic mass or mass number different from the atomic mass or mass number usually found in nature. For example, when hydrogen is present at any position in the compound described herein, it also includes the case where an isotope of hydrogen (e.g., protium, deuterium, tritium, etc.) occurs at that position. Examples of isotopes that can be incorporated into the compounds described herein include, but are not limited to, isotopes of hydrogen, carbon, nitrogen, oxygen, fluorine and chlorine, such as, for example, ²H, ³H, ¹³C, ¹⁴C, ¹⁵N, ¹⁸O, ¹⁷O, ³⁵S, ¹⁸F, ³⁶Cl, respectively. The compounds described herein that comprise certain isotopes (e.g., radioactive isotopes such as ³H and ¹⁴C) are useful in drug and/or substrate tissue distribution assays.

In additional or further embodiments, the compounds described herein are metabolized upon administration to an organism in need to produce a metabolite that is then used to produce a desired effect, including a desired therapeutic effect.

Compounds described herein may be formed as, and/or used as, pharmaceutically acceptable salts. The type of pharmaceutical acceptable salts, include, but are not limited to:(1) acid addition salts, formed by reacting the free base form of the compound with a pharmaceutically acceptable inorganic acid such as hydrochloric acid, hydrobromic acid, sulfuric acid, nitric acid, phosphoric acid, metaphosphoric acid, and the like; or with an organic acid such as acetic acid, propionic acid, hexanoic acid, cyclopentanepropionic acid, glycolic acid, pyruvic acid, lactic acid, malonic acid, adipic acid, sebacic acid, succinic acid, malic acid, maleic acid, fumaric acid, trifluoroacetic acid, tartaric acid, citric acid, benzoic acid, 3-(4-hydroxybenzoyl)benzoic acid, cinnamic acid, mandelic acid, methanesulfonic acid, ethanesulfonic acid, 1,2-ethanedisulfonic acid, 2-hydroxyethanesulfonic acid, benzenesulfonic acid, toluenesulfonic acid, 2-naphthalenesulfonic acid, 4-methylbicyclo-[2.2.2]oct-2-ene-1-carboxylic acid, glucoheptonic acid, hippuric acid, gentisic acid, 4,4'-methylenebis-(3-hydroxy-2-ene-1-carboxylic acid), nicotinic acid, 3-phenylpropionic acid, trimethylacetic acid, tertiary butylacetic acid, lauryl sulfuric acid, gluconic acid, glutamic acid, hydroxynaphthoic acid, salicylic acid, stearic acid, muconic acid, and the like; (2) salts formed when an acidic proton present in the parent compound either is replaced by a metal ion, e.g., an alkali metal ion (e.g. lithium, sodium, potassium), an alkaline earth ion (e.g. magnesium or calcium), or an aluminum ion; or (3) salts formed by coordinates with an organic or inorganic base. Acceptable organic bases include ethanolamine, diethanolamine, triethanolamine, trimethylamine, N-methylglucamine, etc. Acceptable inorganic bases include aluminum hydroxide, calcium hydroxide , potassium hydroxide, sodium carbonate, sodium hydroxide, etc.

The corresponding counterions of the pharmaceutically acceptable salts may be analyzed and identified using various methods including, but not limited to, ion exchange chromatography, ion chromatography, capillary electrophoresis, inductively coupled plasma, atomic absorption spectroscopy, mass spectrometry, or any combination thereof.

The salts can be recovered by using at least one of the following techniques: filtration, precipitation with a non-solvent followed by filtration, evaporation of the solvent, or, in the case of aqueous solutions, lyophilization.

It should be understood that a reference to a pharmaceutically acceptable salt includes the solvent addition forms or crystal forms thereof, particularly solvates or polymorphs. Solvates contain either stoichiometric or non-stoichiometric amounts of a solvent, and may be formed during the process of crystallization with pharmaceutically acceptable solvents such as water, ethanol, and the like. Hydrates are formed when the solvent is water, or alcoholates are formed when the solvent is alcohol. Solvates of the compounds described herein can be conveniently prepared or formed during the processes described herein. In addition, the compounds provided herein can exist in unsolvated as well as solvated forms. In general, the solvated forms are considered equivalent to the unsolvated forms for the purposes of the compounds and methods provided herein.

Compounds described herein may be in various forms, including but not limited to, amorphous forms, milled forms and nano-particulate forms. In addition, compounds described herein include crystalline forms, also known as polymorphs. Polymorphs include the different crystal packing arrangements of the same elemental composition of a compound. Polymorphs usually have different X-ray diffraction patterns, infrared spectra, melting points, density, hardness, crystal shape, optical and electrical properties, stability, and solubility. Various factors such as the recrystallization solvent, rate of crystallization, and storage temperature may cause a single crystal form to dominate.

The screening and characterization of the pharmaceutically acceptable salts, polymorphs and/or solvates may be accomplished using a variety of techniques including, but not limited to, thermal analysis, x-ray diffraction, spectroscopy, vapor sorption, and microscopy. Thermal analysis methods address thermo chemical degradation or thermo physical processes including, but not limited to, polymorphic transitions, and such methods are used to analyze the relationships between polymorphic forms, determine weight loss, to find the glass transition temperature, or for excipient compatibility studies. Such methods include, but are not limited to, Differential scanning calorimetry (DSC), Modulated Differential Scanning Calorimetry (MDCS), Thermogravimetric analysis (TGA), and Thermogravimetric and Infrared analysis (TG/IR). X-ray diffraction methods include, but are not limited to, single crystal and powder diffractometers and synchrotron sources. The various spectroscopic techniques used include, but are not limited to, Raman, FTIR, UVIS, and NMR (liquid and solid state). The various microscopy techniques include, but are not limited to, polarized light microscopy, Scanning Electron Microscopy (SEM) with Energy Dispersive X-Ray Analysis (EDX), Environmental Scanning Electron Microscopy with EDX (in gas or water vapor atmosphere), IR microscopy, and Raman microscopy.

The carrier herein includes conventional diluents, excipients, fillers, binders, wetting agents, disintegrants, absorption enhancers, surfactants, adsorption carriers, lubricants, and, if necessary, flavoring agents, sweeteners or the like, which are commonly used in the field of pharmacy. The medicament of the present invention can be prepared into various forms such as tablets, powders, granules, capsules, oral liquids and injections. The above medicaments in various dosage forms can be prepared by conventional methods in the field of pharmacy.

As used herein, the IC₅₀ refers to an amount, concentration or dosage of a particular test compound that achieves a 50% inhibition of a maximal response, such as inhibition of Btk, in an assay that measures such response.

Throughout the specification, groups and substituents thereof can be chosen by a person skilled in the art to provide stable compounds.

### Examples

The following specific and non-limiting examples are to be construed as merely illustrative, and do not limit the present disclosure in any way whatsoever. Without further elaboration, it is believed that those skilled in the art can, based on the description herein, utilize the present invention to its fullest extent.

Unless specified otherwise, the various compounds, reagents, materials, etc. used in the Examples herein were synthesized or obtained by approaches commonly used by those skilled in the art.

### Synthesis of Compounds

The following abbreviations are used in the following synthesis schemes:
Boc: t-butoxycarbonyl;
BOPCl: Bis(2-oxo-3-oxazolidinyl)sulfinyl chloride;
DCM: dichloromethane;
DIEA: N,N-diisopropylethylamine;
DMAP: 4-dimethylaminopyridine;
DMF: dimethylformamide;
DPB: bis(pinacolato)diboron;
Et: ethyl;
HATU: 2-(7-benzotriazole oxide)-N,N,N',N'-tetramethyluronium hexafluorophosphate;
Me: methyl;
NMP: N-methylpyrrolidone;
Pd(dppf)Cl₂: [1,1'-bis(diphenylphosphino)ferrocene]palladium dichloride;
Pd₂(dba)₃: tris(dibenzylideneacetone)dipalladium;
PMB: p-methoxybenzyl;
rt/RT: room temperature;
TEA: triethylamine;
TFA: trifluoroacetic acid;
THF: tetrahydrofuran;
XantPhos: 4,5-bisdiphenylphosphino-9,9-dimethylxanthene.

### Synthesis Scheme I

### Step 1:

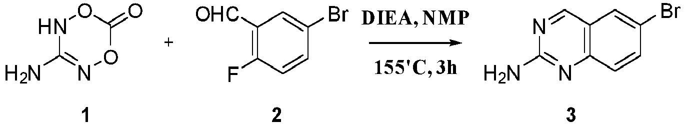

A mixed solution of guanidine carbonate (281 g, 1.56 mol), DIEA (540 mL, 3.12 mol) and NMP (1 L) was heated to 150-160 °C under stirring, and a solution of 5-bromo-2-fluorobenzaldehyde (250 g, 1.20 mol) in NMP (100 ml) was slowly added dropwise. After 3 h reaction, the temperature was decreased to 100 °C. Ice (2 kg) and water (4 L) were added, and a yellow-brown solid was precipitated out. Stirring was continued for 30 min. The reaction was filtered under reduced pressure, and washed with water (1 L) and ethanol (1 L). The resulting filter cake was transferred to a 5 L flask, to which ethanol (2 L) was added. The mixture was stirred for 2 h, filtered, washed with ethanol (0.5 L), toluene/ethanol (1:1, 0.5 L) and toluene (0.5 L) successively, and dried to give Compound 3 as a pale yellow solid (168 g, 48%).

### Step 2:

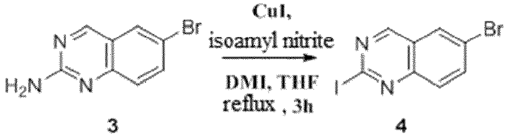

Compound 3 (5.0 g, 22.3 mmol), cuprous iodide (4.30 g, 22.3 mmol), and diiodomethane (9.0 mL, 114 mmol) were dissolved in THF (100 mL). Isoamyl nitrite (9.0 mL, 68 mmol) was added, and heated to reflux for 2.5 h under nitrogen atmosphere. The reaction solution was cooled down to room temperature, and ethyl acetate (500 mL) and 1 N HCl (500 mL) were added. After liquid separation, the aqueous phase was extracted twice with ethyl acetate. The organic phases were combined, washed with a saturated aqueous ammonium chloride solution, dried over anhydrous magnesium sulfate, filtered and concentrated. The resulting oil was separated by column chromatography (dichloromethane, 100%) to give Compound 4 (2.60 g, 35%) as a nearly white powdered solid.

### Step 3:

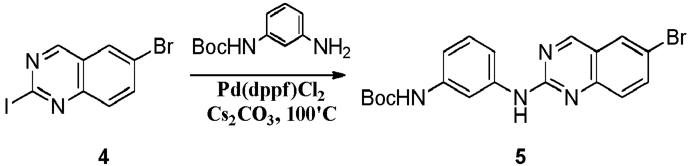

Compound 4 (4.0 g, 12.0 mmol) and *N*-Boc-m-phenylenediamine (3.7 g, 17.9 mmol) were dissolved in 1,4-dioxane (200 mL), and [1,1'-bis(diphenylphosphino)ferrocene]palladium dichloride (820 mg, 1.1 mmol) and cesium carbonate (19.5 g, 60.0 mmol) were added. The reaction system was purged three times with argon, and heated to 100 °C and reacted for 20 h under argon atmosphere. The starting materials were depleted as detected by TLC. The reaction solution was cooled down to room temperature and filtered through celite under reduced pressure. The resulting filtrate was diluted with ethyl acetate and water. After liquid separation, the aqueous phase was extracted twice with ethyl acetate. The organic phases were combined, washed with a saturated saline solution, dried over anhydrous magnesium sulfate, filtered and concentrated. Separation by column chromatography (n-hexane: ethyl acetate = 2:1 to 1:1) obtained Compound 5 (3.4 g, 68%) as a pale yellow powdered solid.

### Step 4:

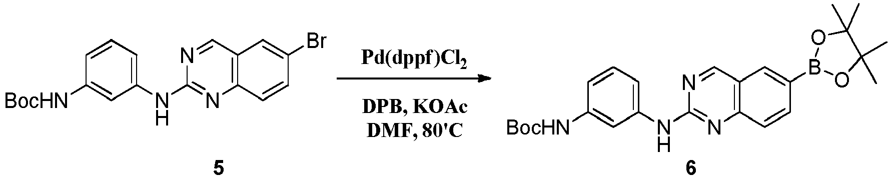

Compound 5 (3.37 g, 8.1 mmol) and bis(pinacolato)diboron (12.17 g, 3.1 mmol) were dissolved in dimethylformamide (200 mL), and [1,1'-bis(diphenylphosphino))ferrocene]palladium dichloride (594 mg, 0.8 mmol) and potassium acetate (4.0 g, 40.5 mmol) were added. The reaction system was purged three times with argon, heated to 80 °C and reacted for 20 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered and concentrated. Separation by column chromatography (n-hexane: ethyl acetate = 10:1 to 5:1) obtained Compound 6 (2.63 g, 70%) as a pale yellow powdered solid.

### Step 5:

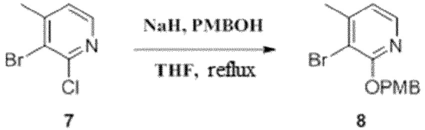

Sodium hydride (3.2 g, 72.7 mmol) was dispersed in 500 mL of tetrahydrofuran. A solution of 4-methoxybenzyl alcohol (10.0 g, 72.4 mmol) in tetrahydrofuran (30 mL) was slowly added while stirring at room temperature. After stirring at room temperature for 30 min, 3-bromo-2-chloro-4-methylpyridine (10.0 g, 48.4 mmol) was added to the solution. The reaction system was heated to 75 °C and stirred for 5 h. After the reaction was completed, it was quenched with water (50 mL), and diluted with ethyl acetate. After liquid separation, the organic phase was washed with water and saturated brine successively. The final organic phase was dried over anhydrous magnesium sulfate, concentrated under reduced pressure, and purified by silica gel column chromatography (n-hexane: ethyl acetate = 50:1) to give Compound 8 (12.0 g, 81%) as a pale yellow viscous liquid.

### Step 6:

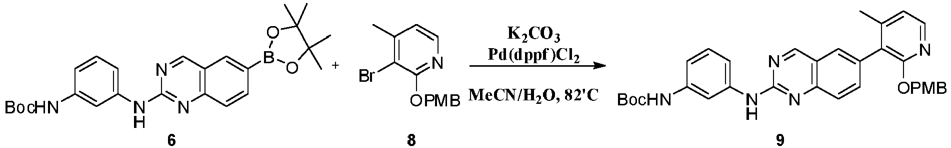

Compound 6 (230 mg, 0.50 mmol) and Compound 8 (230 mg, 0.75 mmol) were dissolved in a mixed solution of acetonitrile and water (60 mL/20 mL), and [1,1'-bis(diphenylphosphino)ferrocene] palladium dichloride (37 mg, 0.05 mmol) and potassium carbonate (414 mg, 3.00 mmol) were added. The reaction system was purged three times with argon, and heated to 82 °C and reacted for 20 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered and concentrated. Separation by column chromatography (n-hexane: ethyl acetate = 2:1 to 1:1) obtained Compound 9 (200 mg, 71%) as a yellow powdered solid.

### Step 7:

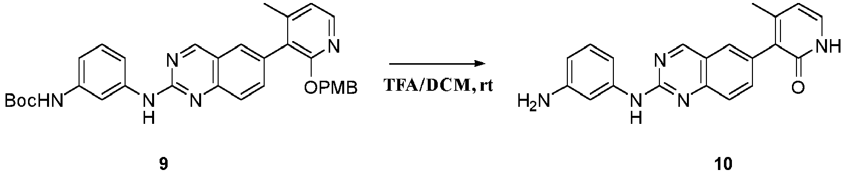

Compound 9 (550 mg, 0.98 mmol) was dispersed in 100 mL of dichloromethane. 25 mL of trifluoroacetic acid was slowly dropped into the reaction system while stirring. After the final reaction system was constantly stirred at room temperature for 2 h, it was concentrated under reduced pressure to give a solid. The residue was dissolved in ethyl acetate and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 15:1) to afford Compound 10 (264 mg, 79%) as a pale yellow solid.

### Synthesis Scheme II

### Method 1:

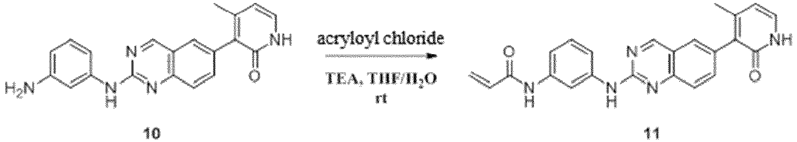

Compound 10 (430 mg, 1.25 mmol) was dispersed in a mixed solvent of THF and water (200 mL, 4: 1 V/V), and then triethylamine (0.26 mL, 1.87 mmol) was added. While slow stirring, acryloyl chloride (150 µL, 1.87 mmol) was slowly dropped into the reaction system. After the reaction solution was stirred at room temperature for 2 h, it was concentrated under reduced pressure. The residue was dissolved in ethyl acetate and washed with a 10% citric acid solution and saturated brine successively. The final organic phase was dried over anhydrous magnesium sulfate and concentrated under reduced pressure. The concentrate was purified by silica gel column chromatography (dichloromethane: methanol = 10:1) to afford Compound 11 (367 mg, yield: 92%) as a pale yellow powdered solid.

### Method 2:

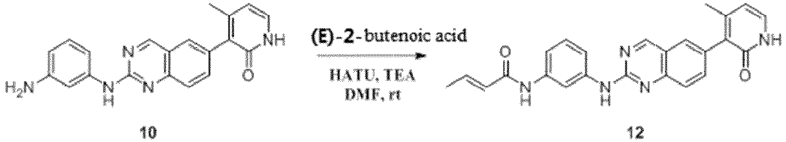

Compound 10 (25 mg, 0.07 mmol) and 2-butenoic acid (7 mg, 0.08 mmol) were dissolved in dimethylformamide (5 mL), and then HATU (40 mg, 0.11 mmol) was added. While slow stirring, triethylamine (30 µL, 0.22 mmol) was slowly dropped into the reaction system. The reaction solution was heated to 70 °C, and stirred for 3 h. The starting materials were depleted as detected by TLC. After concentration under reduced pressure, the residue was dissolved in ethyl acetate and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 25:1) to afford Compound 12 (15 mg, yield: 52%) as a pale yellow powdered solid.

### Method 3:

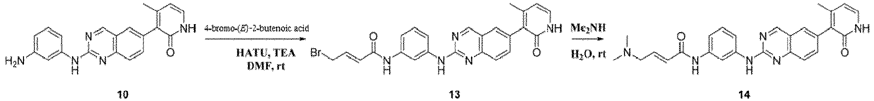

Compound 10 (1.10 g, 3.21 mmol) and 4-bromo-2-trans-butenoic acid (0.69 g, 4.14 mmol) were dissolved in dimethylformamide (10 mL), and then HATU (2.20 g, 5.78 mmol) was added. While slow stirring, triethylamine (1.3 mL, 9.63 mmol) was slowly dropped into the reaction system. The reaction solution was stirred at room temperature for 3 h. The starting materials were depleted as detected by TLC. After concentration under reduced pressure, the residue was dissolved in ethyl acetate and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 20:1) to afford Compound 13 (1.10 g, yield: 70%) as a pale yellow powdered solid.

Compound 13 (146 mg, 0.3 mmol) was dissolved in THF (5 mL), and then DIPEA (98 µL, 0.6 mmol) was added. While slow stirring, a dimethylamine solution (67 µL, 0.6 mmol) was slowly dropped into the reaction system. The reaction solution was stirred at room temperature overnight and concentrated under reduced pressure. The residue was dissolved in ethyl acetate and washed with a 10% citric acid solution and saturated brine successively. The final organic phase was dried over anhydrous magnesium sulfate and concentrated under reduced pressure. The concentrate was purified by silica gel column chromatography (dichloromethane: methanol = 10:1) to afford Compound 14 (84 mg, yield: 62%) as a pale yellow powdered solid.

### Synthesis Scheme III

### Step 1:

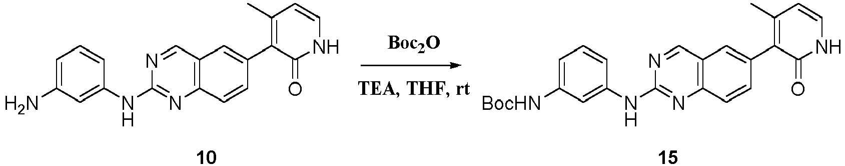

Compound 10 (90 mg, 0.26 mmol) was dispersed in a mixed solvent of THF and water (30 mL, 1:1 V/V), and then triethylamine (62 µL, 0.45 mmol) was added. While stirring, Boc₂O (80 µL, 0.35 mmol) was slowly dropped into the reaction system at 0 °C. After the reaction solution was stirred at room temperature for 24 h, ethyl acetate was added for dissolution, followed by liquid separation. The organic phase was washed with a saturated sodium bicarbonate solution and saturated brine successively, dried over anhydrous magnesium sulfate, and concentrated under reduced pressure. The concentrate was purified by silica gel column chromatography (dichloromethane: methanol = 20:1) to give Compound 15 (61 mg, yield: 53%) as a pale yellow solid.

### Step 2:

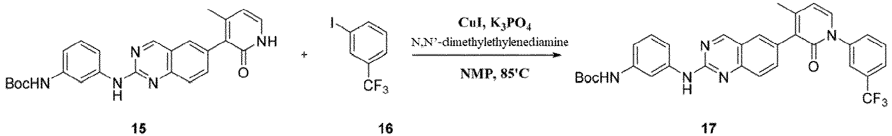

Compound 15 (61 mg, 0.14 mmol), potassium phosphate (117 mg, 0.55 mmol), and cuprous iodide (11 mg, 0.06 mmol) were dispersed in NMP (5 mL), and Compound 16 (40 µL, 0.28 mmol) and N,N'-dimethylethylenediamine (15 µL, 0.14 mmol) were added. The reaction system was purged three times with argon, heated to 85 °C and reacted for 6 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered, concentrated, and separated by column chromatography (n-hexane: ethyl acetate = 5:1 to 2:1) to give Compound 17 (84 mg, 99%) as a yellow-green powdered solid.

### Step 3:

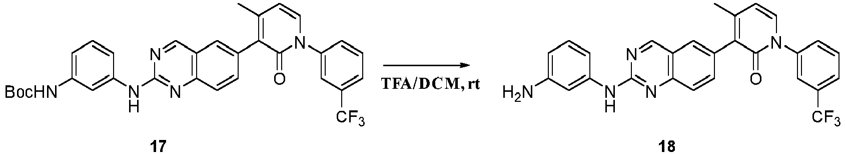

Compound 17 (84 mg, 0.14 mmol) was dispersed in 25 mL of dichloromethane, and 5 mL of trifluoroacetic acid was slowly dropped into the reaction system while stirring. After the final reaction system was constantly stirred at room temperature for 1 h, it was concentrated under reduced pressure to give a solid. The residue was dissolved in ethyl acetate and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 50:1) to give Compound 18 (30 mg, 43%) as a yellow powdered solid.

### Step 4:

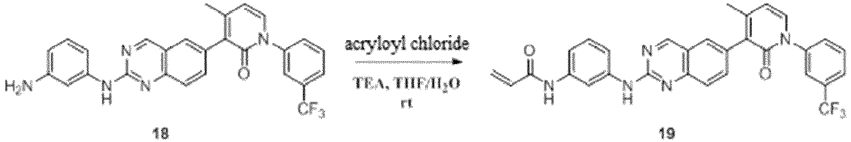

Compound 18 (23 mg, 0.05 mmol) was dispersed in a mixed solvent of THF and water (15 mL, 3:1 V/V), and then triethylamine (10 µL, 0.07 mmol) was added. While slow stirring, acryloyl chloride (6 µL, 0.07 mmol) was slowly dropped into the reaction system. After the reaction solution was stirred at room temperature for 3 h, it was concentrated under reduced pressure. The residue was dissolved in ethyl acetate and washed with a 10% citric acid solution and saturated brine successively. The final organic phase was dried over anhydrous magnesium sulfate and concentrated under reduced pressure. The concentrate was purified by silica gel column chromatography (dichloromethane: methanol = 50:1) to afford Compound 19 (17 mg, yield: 67%) as a pale yellow-green powdered solid.

### Synthesis Scheme IV

### Step 1:

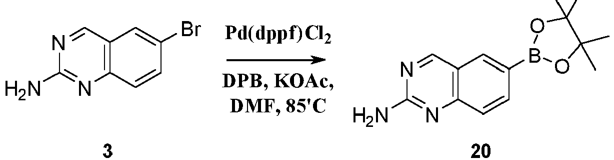

Compound 3 (500 mg, 2.25 mmol) and bis(pinacolato)diboron (860 mg, 3.38 mmol) were dissolved in dimethylformamide (50mL), and [1,1'-bis(diphenylphosphino)ferrocene]palladium dichloride (165 mg, 0.23 mmol) and potassium acetate (1.1 g, 11.25 mmol) were added. The reaction system was purged three times with argon, heated to 85 °C and reacted for 20 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered, concentrated, and separated by column chromatography (n-hexane: ethyl acetate = 5:1 to 1:1) to give Compound 20 (310 mg, 51%) as a pale yellow powdered solid.

### Step 2:

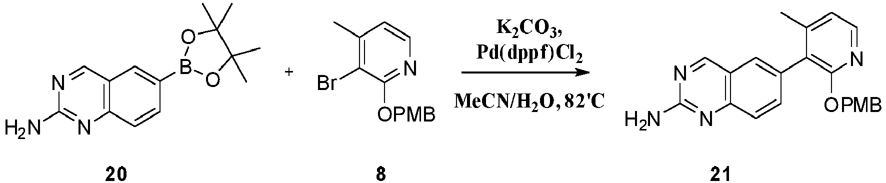

Compound 20 (140 mg, 0.5 mmol) and Compound 8 (230 mg, 0.75 mmol) were dissolved in a mixed solution of acetonitrile and water (40 mL/15 mL), and [1,1'-bis(diphenylphosphino)ferrocene]palladium dichloride (37 mg, 0.05 mmol) and potassium carbonate (414 mg, 3.00 mmol) were added. The reaction system was purged three times with argon, and heated to 82 °C and reacted for 20 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered, concentrated, and separated by column chromatography (n-hexane: ethyl acetate = 2:1 to 1:2) to give Compound 21 (111 mg, 60%) as a yellow powdered solid.

### Step 3:

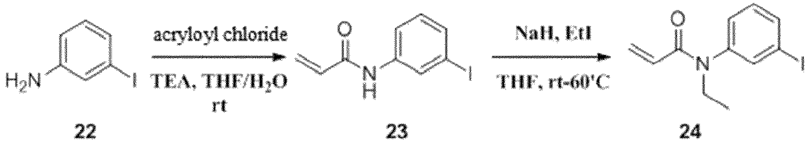

Compound 22 (2.0 g, 22.83 mmol) was dispersed in a mixed solvent of THF and water (250 mL, 4:1 V/V), and then triethylamine (4.8 mL, 34.25 mmol) was added. While slow stirring, acryloyl chloride (2.8 mL, 34.25 mmol) was slowly dropped into the reaction system. After the reaction solution was stirred at room temperature for 12 h, it was concentrated under reduced pressure. The residue was dissolved in ethyl acetate and washed with a 10% citric acid solution and saturated brine successively. The final organic phase was dried over anhydrous magnesium sulfate and concentrated under reduced pressure to give compound 23 (6.17 g, crude yield: 99%) as a nearly white powdered solid.

60% sodium hydride (1.5g, 37.5 mmol) was dispersed in 50 mL of tetrahydrofuran, and Compound 23 (1.5 g, 5.5 mmol) was slowly added while stirring at 0 °C. The reaction was warmed to room temperature and stirred for 30 min. Iodoethane (1.5 mL, 18.7 mmol) was added dropwise to the solution. The reaction system was heated to 60 °C and stirred for 20 h. After the reaction was completed, it was quenched with water (50 mL) and diluted with ethyl acetate. After liquid separation, the organic phase was washed with water and saturated brine successively. The final organic phase was dried over anhydrous magnesium sulfate, concentrated under reduced pressure, and purified by silica gel column chromatography (n-hexane: ethyl acetate = 4:1) to give Compound 24 (1.2 g, 70%) as a deep yellow viscous liquid.

### Step 4:

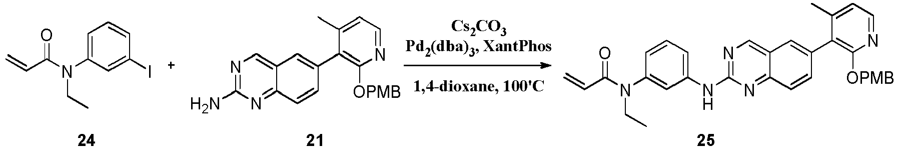

Compound 24 (1.2 g, 4.0 mmol) and Compound 21 (1.0 g, 2.7 mmol) were dissolved in 1,4-dioxane (100 mL), and Pd₂(dba)₃ (250 mg, 0.27 mmol), XantPhos ligand (312 mg, 0.54 mmol) and cesium carbonate (4.4 g, 13.5 mmol) were added. The reaction system was purged three times with argon, and heated to 82 °C and reacted for 20 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered, concentrated, and separated by column chromatography (n-hexane: ethyl acetate = 2:1 to 1:2) to give Compound 25 (840 mg, 57%) as a yellow powdered solid.

### Step 5:

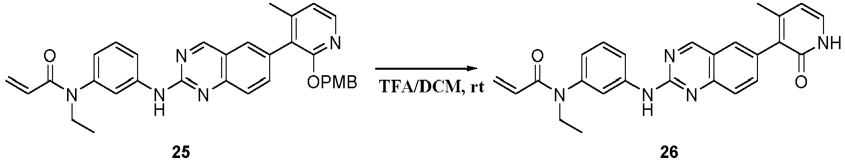

Compound 25 (100 mg, 0.18 mmol) was dispersed in 25 mL of dichloromethane, and 5 mL of trifluoroacetic acid was slowly dropped into the reaction system while stirring. After the final reaction system was constantly stirred at room temperature for 1 h, it was concentrated under reduced pressure to give a solid. The residue was dissolved in ethyl acetate, and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 50:1) to afford Compound 26 (31 mg, 40%) as a pale yellow powdered solid.

### Synthesis Scheme V

### Method 1:

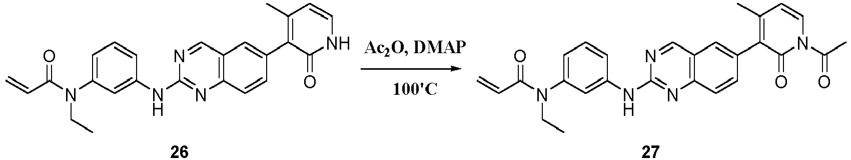

Compound 26 (30 mg, 0.07 mmol) was dissolved in acetic anhydride (10 mL), and 4-dimethylaminopyridine (8 mg) was added. The reaction system was heated to 100 °C for 2 h. The starting materials were depleted as detected by TLC. The reaction solution was cooled down to room temperature, spun to remove the solvent under reduced pressure, and diluted with ethyl acetate and water. After liquid separation, the aqueous phase was extracted twice with ethyl acetate. The organic phases were combined, washed with a saturated sodium bicarbonate solution and saturated saline solution, dried over anhydrous magnesium sulfate, filtered, concentrated, and separated by column chromatography (n-hexane: ethyl acetate = 2:1 to 1:2) to afford Compound 27 (14 mg, 43%) as a nearly white powdered solid.

### Method 2:

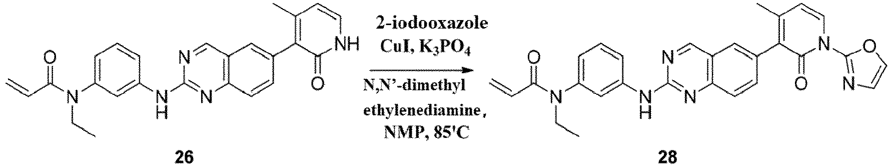

Compound 26 (50 mg, 0.12 mmol) was dissolved in NMP (5 mL), and cuprous iodide (10 mg, 0.05 mmol), potassium phosphate (100 mg, 0.50 mmol), 2-iodooxazole (50 mg, 0.24 mmol), and N,N'-dimethylethylenediamine (15 µL, 0.12 mmol) were added successively. The reaction system was heated to 85 °C for 24 h. The starting materials were depleted as detected by TLC. The reaction solution was cooled down to room temperature, spun to remove the solvent under reduced pressure, and diluted with ethyl acetate and water. After liquid separation, the aqueous phase was extracted twice with ethyl acetate. The organic phases were combined, washed with a saturated sodium bicarbonate solution and a saturated saline solution, dried over anhydrous magnesium sulfate, filtered, concentrated, and separated by column chromatography (dichloromethane: methanol = 25:1) to afford Compound 28 (30 mg, 51%) as a yellow powdered solid.

### Synthesis Scheme VI

### Step 1:

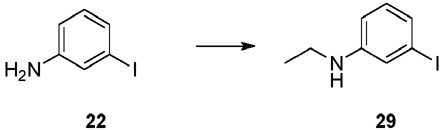

60% sodium hydride (6.0 g, 150 mmol) was dispersed in 300 mL of tetrahydrofuran, and Compound 22 (6.6 g, 30 mmol) was slowly added under stirring at 0 °C. The reaction was warmed to room temperature and stirred for 30 min. Iodoethane (3.6 mL, 45 mmol) was added to the solution. The reaction system was heated to 60 °C and stirred for 20 h. After the reaction was completed, it was quenched with water (50 mL) and diluted with ethyl acetate. After liquid separation, the organic phase was washed with water and saturated brine successively. The final organic phase was dried over anhydrous magnesium sulfate, concentrated under reduced pressure, and purified by silica gel column chromatography (n-hexane: ethyl acetate = 4:1) to give Compound 29 (5.2 g, 70%) as a deep yellow viscous liquid.

### Step 2:

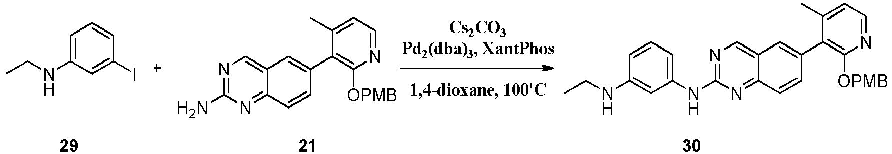

Compound 29 (470 mg, 1.9 mmol) and Compound 21 (590 mg, 1.6 mmol) were dissolved in 1,4-dioxane (50 mL), and Pd₂(dba)₃ (147 mg, 0.20 mmol), XantPhos ligand (185 mg, 0.40 mmol), and cesium carbonate (2.6 g, 8.0 mmol) were added. The reaction system was purged three times with argon, heated to 100 °C and reacted for 20 h under argon atmosphere. The starting materials were depleted as detected by TLC. The reaction solution was cooled down to room temperature and filtered through celite. The resulting filtrate was spun to remove the solvent under reduced pressure, and diluted with ethyl acetate and water. After liquid separation, the aqueous phase was extracted twice with ethyl acetate. The organic phases were combined, washed with a saturated saline solution, dried over anhydrous magnesium sulfate, filtered, concentrated, and separated by column chromatography (n-hexane: ethyl acetate = 2:1 to 1:1) to give Compound 30 (276 mg, 56%) as a yellow powdered solid.

### Step 3:

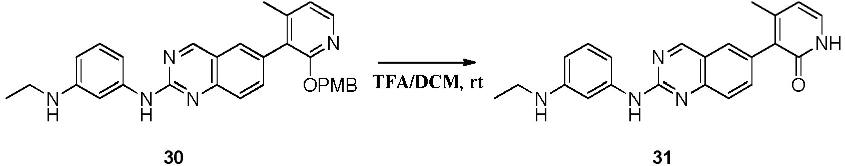

Compound 30 (84 mg, 0.17 mmol) was dispersed in 10 mL of dichloromethane, and 1 mL of trifluoroacetic acid was slowly dropped into the reaction system while stirring. After the final reaction system was constantly stirred at room temperature for 1 h, it was concentrated under reduced pressure to give a solid. The residue was dissolved in ethyl acetate and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 50:1) to afford Compound 31 (42 mg, 66%) as a yellow powdered solid.

### Synthesis Scheme VII

### Step 1:

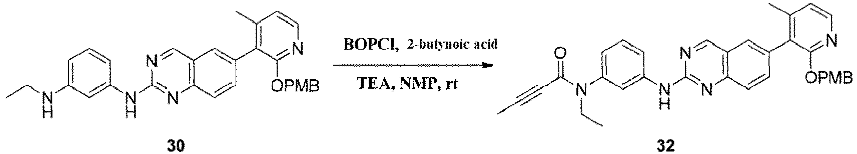

Compound 30 (50 mg, 0.10 mmol) and 2-butynoic acid (51 mg, 0.60 mmol) were dissolved in NMP (10 mL), and then BOPC1 (150 mg, 0.60 mmol) was added. The reaction solution was stirred at room temperature for 3 h. The starting materials were depleted as detected by TLC. A saturated sodium bicarbonate solution was added to the reaction system, which was extracted with ethyl acetate. The organic phase was washed with saturated brine, dried over anhydrous magnesium sulfate, and concentrated under reduced pressure. The crude Compound 32 was directly used in the next step.

### Step 2:

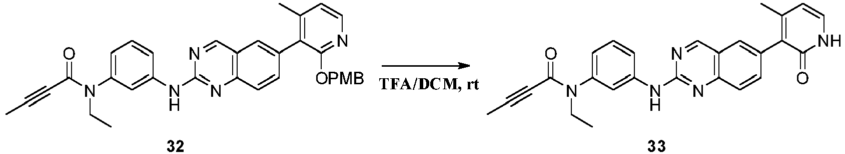

The crude Compound 32 from step 1 was dispersed in 6 mL of dichloromethane, and 1.5 mL of trifluoroacetic acid was slowly dropped into the reaction system while stirring. After the final reaction system was constantly stirred at room temperature for 1 h, it was concentrated under reduced pressure to give a solid. The residue was dissolved in ethyl acetate and washed with a saturated sodium bicarbonate solution and saturated brine successively. The final organic phase was dried over anhydrous magnesium sulfate and concentrated under reduced pressure. The concentrate was purified by silica gel column chromatography (dichloromethane: methanol = 15:1) to afford Compound 33 (16 mg, two-step yield 34%) as a yellow powdered solid.

### Test Example 1: Analysis of In vitro inhibitory activity of Btk

In a cell-free kinase assay, the method described below or a similar method was used to determine the half inhibitory concentration (IC₅₀) of the compound of the present invention on Btk.

Btk kinase activity was determined using a time-resolved fluorescence resonance energy transfer (TR-FRET) methodology. Measurements were performed in a reaction volume of 50 µL using 96-well assay plates. Kinase, inhibitor, ATP (at the Kₘ for the kinase), and 1 µM peptide substrate (Biotin-AVLESEEELYSSARQ-NH₂) were incubated in a reaction buffer consisting of 20 mM Tris, 50 mM NaCl, MgCl₂ (5-25 mM, depending on the kinase), MnCl₂ (0-10 mM), 1 mM DTT, 0.1 mM EDTA, 0.01% bovine serum albumin, 0.005% Tween-20, and 10% DMSO at pH 7.4 for 1 h. The reaction was quenched by the addition of 1.2 equivalents of EDTA (relative to divalent cation) in 25 µL of 1x Lance buffer (Perkin-Elmer). Streptavidin-APC (Perkin-Elmer) and Eu-labeled p-Tyr100 antibody (Perkin-Elmer) in 1x Lance buffer were added in a volume of 25 µL to give final concentrations of 100 nM and 2.5 nM, respectively. The mixture was allowed to incubate for 1 h. The TR-FRET signal was measured on a multimode plate reader at an excitation wavelength (λ_{Ex}) of 330 nm and detection wavelengths (λ_{Em}) of 615 and 665 nm. The activity was determined by the ratio of fluorescence at 665 nm to that at 615 nm. For each compound, the enzyme activity was measured in the presence of different concentrations of the compound. Negative control reactions were performed in the absence of inhibitor in replicates of six, and two no-enzyme controls were used to determine baseline fluorescence levels. IC₅₀ was obtained by fitting using the program Batch Kᵢ (Kuzmic, et al. (2000), Anal. Biochem. 286:45-50).

The Example compounds 1-50 of the present invention were synthesized according to the above Synthetic Schemes I to VII. The specific synthesis schemes and characterization of the Example compounds are shown in Table 1 below. In the analysis of *in vitro* inhibitory activity of Btk, the IC₅₀ values of the Example compounds 1-50 of the present invention were determined. The IC₅₀ values are given according to the interval of the IC₅₀ value, where "+++" means IC₅₀ < 100 nM; "++" means 100 nM ≤ IC₅₀ < 1000 nM; and "+" means 1000 nM ≤ IC₅₀ < 10000 nM.

**Table 1. Synthesis and Btk IC₅₀ values of Example compounds**

| Ex. | Structure | Synthesis Scheme | Data of structure | Effic acy |
|---|---|---|---|---|
| **1** | 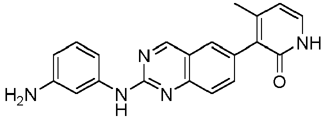 | Synthesized according to Synthesis Scheme I | HRMS (ESI) m/z [M+H]⁺ calc'd for C20H18N5O: 344.1511, found: 344.1505 | +++ |
| **2** | 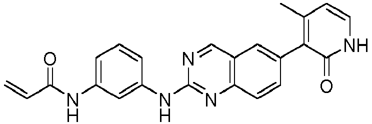 | Synthesized according to Synthesis Scheme II, method 1 | HRMS (ESI) m/z [M+H]⁺ calc'd for C23H20N5O2: 398.1617, found: 398.1613 | +++ |
| **3** | 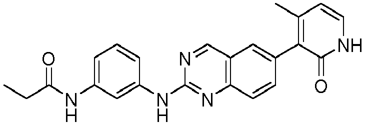 | Synthesized according to Synthesis Scheme II, method 1, except using propionyl chloride instead of acryloyl chlorid e | HRMS (ESI) m/z [M+H]⁺ calc'd for C23H22N5O2: 400.1773, found: 400.1769 | +++ |
| **4** | 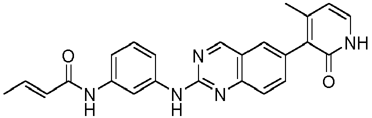 | Synthesized according to Synthesis Scheme II, method 2 | HRMS (ESI) m/z [M+H]⁺ calc'd for C24H22N5O2: 412.1773, found: 402.1752 | +++ |
| **5** | 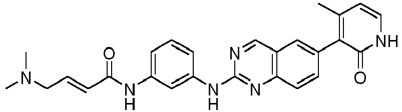 | Synthesized according to Synthesis Scheme II, method 3 | HRMS (ESI) m/z [M+H]⁺ calc'd for C26H27N6O2: 455.2195, found: 455.2189 | +++ |
| **6** | 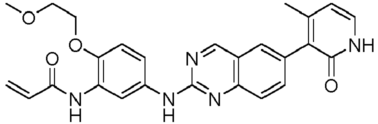 | Synthesized according to Synthesis Scheme I and Synthesis Scheme II, method 2, except using 4-(2-methoxyethoxy )-1,3-m-phenylenediami ne instead of 1,3-m-phenylenediami ne | HRMS (ESI) m/z [M+H]⁺ calc'd for C26H26N5O4: 472.1985, found: 472.1979 | +++ |
| **7** | 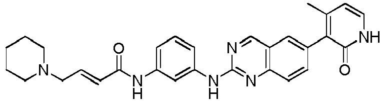 | Synthesized according to Synthesis Scheme II, method 3, except using piperazine instead of dimethylamine | HRMS (ESI) m/z [M+H]⁺ calc'd for C29H31N6O2: 495.2508, found: 495.2503 | +++ |
| **8** | 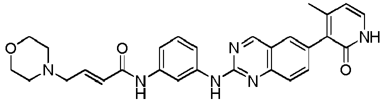 | Synthesized according to Synthesis Scheme II, method 3, except using morpholine instead of dimethy lamine | HRMS (ESI) m/z [M+H]⁺ calc'd for C28H29N6O3 : 497.2301, found: 497.2294 | +++ |
| **9** | 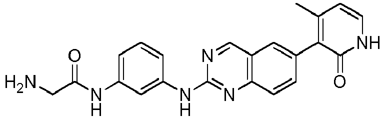 | Synthesized according to Synthesis Scheme II, method 2, except using N-Boc-glycine instead of 2-butenoic acid | HRMS (ESI) m/z [M+H]⁺ calc'd for C22H21N6O2: 401.1726, found: 401.1723 | +++ |
| **10** | 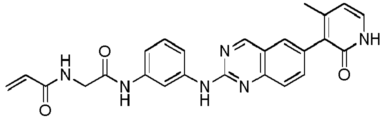 | Synthesized according to Synthesis Scheme II, method 1 | HRMS (ESI) m/z [M+H]⁺ calc'd for C25H23N6O3 : 455.1832, found: 455.1835 | +++ |
| **11** | 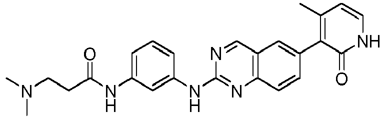 | Synthesized according to Synthesis Scheme II, method 2, except using 3-dimethy lamine acrylic acid instead of 2-butenoic acid | HRMS (ESI) m/z [M+H]⁺ calc'd for C25H27N6O2: 443.2195, found: 443.2191 | +++ |
| **12** | 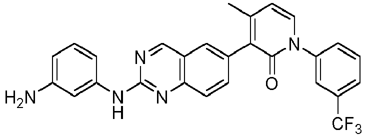 | Synthesized according to Synthesis Scheme III | HRMS (ESI) m/z [M+H]⁺ calc'd for C27H21N5OF3: 488.1698, found: 488.1691 | + |
| **13** | 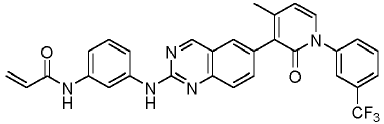 | Synthesized according to Synthesis Scheme III | HRMS (ESI) m/z [M+H]⁺ calc'd for C30H23N5O2F3: 542.1804, found: 542.1830 | ++ |
| **14** | 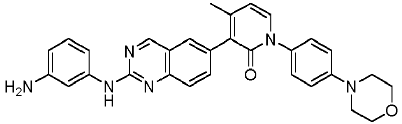 | Synthesized according to Synthesis Scheme III, except using 4-(4-iodophenyl)mor pholine instead of 3-trifluoromethyl iodobenzene in Step 2 | HRMS (ESI) m/z [M+H]⁺ calc'd for C30H29N6O2: 505.2352, found: 505.2348 | ++ |
| **15** | 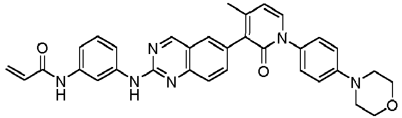 | Synthesized according to Synthesis Scheme III, except using 4-(4-iodophenyl)mor pholine instead of 3-trifluoromethyl iodobenzene in Step 2 | HRMS (ESI) m/z [M+H]⁺ calc'd for C33H31N6O3: 559.2458, found: 505.2444 | ++ |
| **16** | 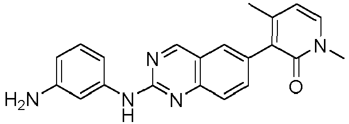 | Synthesized according to Synthesis Scheme I, except using 3-bromo-1,4-dimethylpyridin -2(1H)-one instead of 3-bromo-2-(4-methoxybenzyl oxy)-4-methyl pyridine | HRMS (ESI) m/z [M+H]⁺ calc'd for C21H20N5O: 358.1668, found: 358.1664 | + |
| **17** | 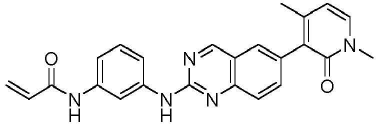 | Synthesized according to Synthesis Scheme I and Synthesis Scheme II, method 1, except using 3-bromo-1,4-dimethylpyridin -2(1H)-one instead of 3-bromo-2-(4-methoxybenzyl oxy)-4-methyl pyridine | HRMS (ESI) m/z [M+H]⁺ calc'd for C24H22N5O: 412.1773, found: 412.1782 | ++ |
| **18** | 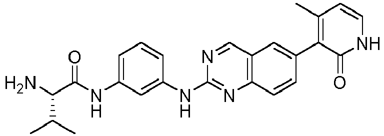 | Synthesized according to Synthesis Scheme II, method 2, except using N-Boc-L-valine instead of 2-butenoic acid | HRMS (ESI) m/z [M+Na]+ calc'd for C25H26N6O2Na: 465.2015, found: 465.2020 | +++ |
| **19** | 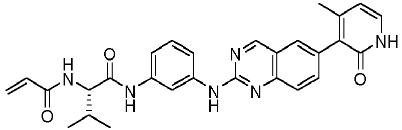 | Synthesized according to Synthesis Scheme II, method 1 | HRMS (ESI) m/z [M+H]⁺ calc'd for C28H29N6O3: 497.2301, found: 497.2302 | +++ |
| **20** | 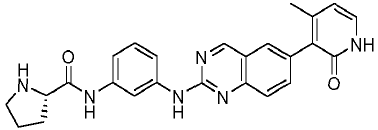 | Synthesized according to Synthesis Scheme II, method 2, except using N-Boc-L-proline instead of 2-butenoic acid | HRMS (ESI) m/z [M+H]⁺ calc'd for C25H25N6O2: 441.2039, found: 441.2043 | ++ |
| **21** | 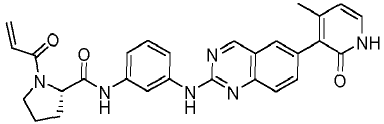 | Synthesized according to Synthesis Scheme II, method 2 | HRMS (ESI) m/z [M+H]⁺ calc'd for C28H27N6O3: 495.2145, found: 495.2182 | +++ |
| **22** | 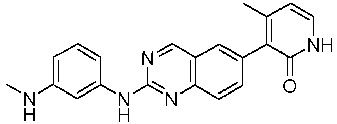 | Synthesized according to Synthesis Scheme VI, except using iodomethane instead of iodoethane in Step 1 | HRMS (ESI) m/z [M+H]⁺ calc'd for C21H20N5O: 358.1668, found: 358.1682 | +++ |
| **23** | 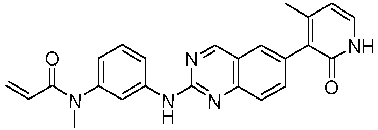 | Synthesized according to Synthesis Scheme IV, except using iodomethane instead of iodoethane in Step 3 | HRMS (ESI) m/z [M+H]⁺ calc'd for C24H22N5O2: 412.1773, found:412.1796 | +++ |
| **24** | 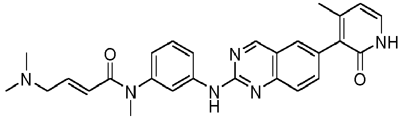 | Synthesized according to Synthesis Scheme II, method 3 | HRMS (ESI) m/z [M+H]⁺ calc'd for C27H29N6O2: 496.2352, found: 496.2350 | +++ |
| **25** | 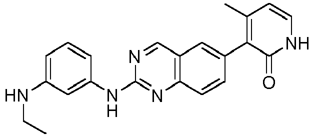 | Synthesized according to Synthesis Scheme VI | HRMS (ESI) m/z [M+H]⁺ calc'd for C22H22N5O: 372.1824, found: 372.1826 | +++ |
| **26** | 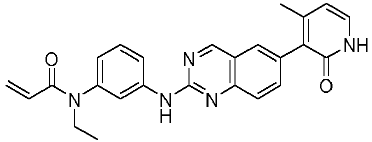 | Synthesized according to Synthesis Scheme IV | HRMS (ESI) m/z [M+H]⁺ calc'd for C25H24N5O2: 426.1930, found: 426.1929 | +++ |
| **27** | 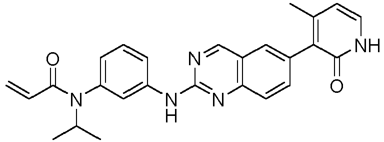 | Synthesized according to Synthesis Scheme IV, except using iodoisopropane instead of iodoethane in Step 3 | HRMS (ESI) m/z [M+H]⁺ calc'd for C26H26N5O2: 440.2087, found: 440.2081 | +++ |
| **28** | 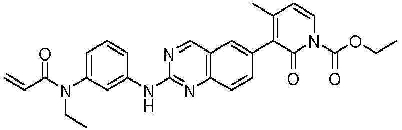 | Synthesized according to Synthesis Scheme V, except using ethoxy carbonyl chloride instead of acetic anhydride in the step | HRMS (ESI) m/z [M+H]⁺ calc'd for C28H28N5O4: 498.2141, found: 498.2137 | +++ |
| **29** | 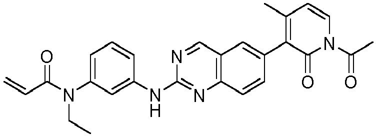 | Synthesized according to Synthesis Scheme V | HRMS (ESI) m/z [M+H]⁺ calc'd for C27H26N5O3: 468.2036, found: 468.2025 | ++ |
| **30** | 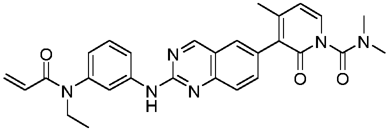 | Synthesized according to Synthesis Scheme V, except using dimethy lamino formyl chloride instead of acetic anhydride in the step | HRMS (ESI) m/z [M+H]⁺ calc'd for C28H29N6O3: 497.2301, found: 497.2296 | ++ |
| **31** | 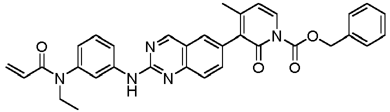 | Synthesized according to Synthesis Scheme V, except using benzyloxycarbo nyl chloride instead of acetic anhydride in the step | HRMS (ESI) m/z [M+H]⁺ calc'd for C33H30N5O4: 560.2298, found: 560.2295 | +++ |
| **32** | 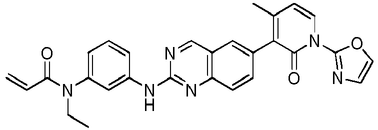 | Synthesized according to Synthesis Scheme V, method 2 | HRMS (ESI) m/z [M+H]⁺ calc'd for C28H25N6O3: 493.1988, found: 493.1993 | ++ |
| **33** | 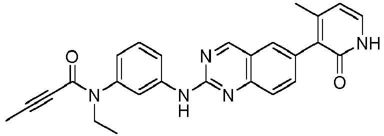 | Synthesized according to Synthesis Scheme VII | HRMS (ESI) m/z [M+H]⁺ calc'd for C26H24N5O2: 438.1930, found: 438.1921 | +++ |
| **34** | 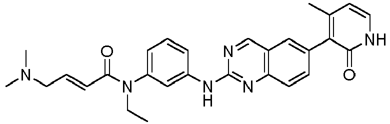 | Synthesized according to Synthesis Scheme VII, except using 4-dimethy lamino 2-butenoic acid instead of 2-butynoic acid | HRMS (ESI) m/z [M+H]⁺ calc'd for C28H31N6O2: 483.2508, found: 483.2516 | +++ |
| **35** | 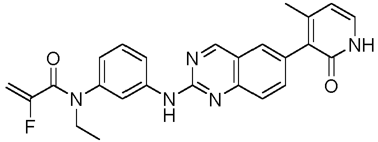 | Synthesized according to Synthesis Scheme VII, except using 2-fluoro-2-acrylic acid instead of 2-butynoic acid | HRMS (ESI) m/z [M+H]⁺ calc'd for C25FH23N5O2: 444.1836, found: 444.1831 | +++ |
| **36** | 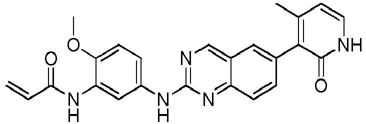 | Synthesized according to Synthesis Scheme I 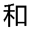 Synthesis Scheme II, method 2, except using 4-methoxy -1,3-m-phenylenediami ne instead of 1,3-m-phenylenediami ne | HRMS (ESI) m/z [M+H]⁺ calc'd for C24H22N5O3: 428.1723, found: 428.1738 | +++ |
| **37** | 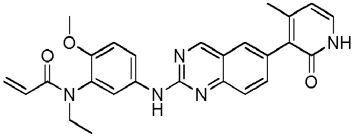 | Synthesized according to Synthesis Scheme IV, except using 2-methoxy-5-iodoaniline instead of 3-iodoaniline | HRMS (ESI) m/z [M+H]⁺ calc'd for C26H26N5O3: 456.2036, found: 456.2031 | +++ |
| **38** | 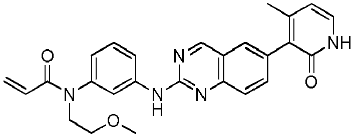 | Synthesized according to Synthesis Scheme IV, except using 2-methoxy-1-iodoethane instead of iodoethane | HRMS (ESI) m/z [M+H]⁺ calc'd for C26H26N5O3: 456.2036, found: 456.2037 | +++ |
| **39** | 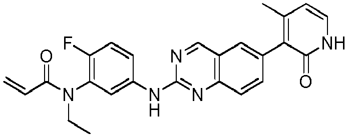 | Synthesized according to Synthesis Scheme IV, except using 2-fluoro-5-iodoaniline instead of 3-iodoaniline | HRMS (ESI) m/z [M+H]⁺ calc'd for C25H23N5O2F: 444.1836, found: 444.1834 | +++ |
| **40** | 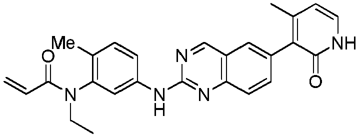 | Synthesized according to Synthesis Scheme IV, except using 2-methyl-5-iodoaniline instead of 3-iodoaniline | HRMS (ESI) m/z [M+H]⁺ calc'd for C26H26N5O2: 440.2087, found: 440.2101 | +++ |
| **41** | 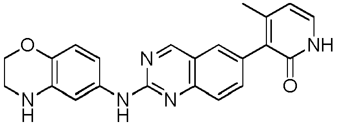 | Synthesized according to Synthesis Scheme I, except using 2, 3-dihydro-2H-benzo[b][1,4]-oxazine-6-amine instead of 1,3-m-phenylenediami ne | HRMS (ESI) m/z [M+H]⁺ calc'd for C22H20N5O2: 386.1637, found: 386.1612 | +++ |
| **42** | 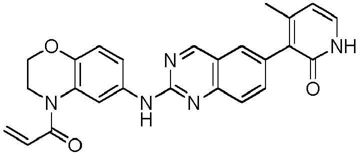 | Synthesized according to Synthesis Scheme I and Synthesis Scheme II, method 2, except using 2, 3-dihydro-2H-benzo[b][1,4]-oxazine-6-amine instead of 1,3-m-phenylenediami ne | HRMS (ESI) m/z [M+H]⁺ calc'd for C25H22N5O3: 440.1723, found: 440.1710 | +++ |
| **43** | 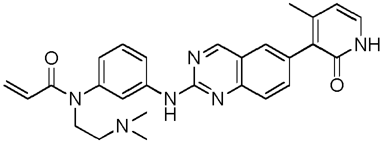 | Synthesized according to Synthesis Scheme IV, except using 2-dimethy lamino-1-iodoethane instead of iodoethane | HRMS (ESI) m/z [M+H]⁺ calc'd for C27H29N6O2: 469.2352, found: 469.2349 | +++ |
| **44** | 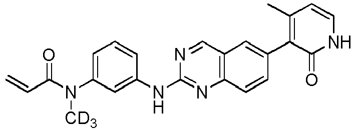 | Synthesized according to Synthesis Scheme IV, except using d3-iodomethane instead of iodoethane in Step 3 | HRMS (ESI) m/z [M+H]⁺ calc'd for C24H19D3N5O2: 415.1962, found: 415.1962 | +++ |
| **45** | 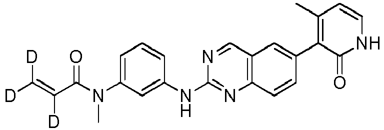 | Synthesized according to Synthesis Scheme IV, except using iodomethane instead of iodoethane, and using d3-acryloyl chloride instead of acryloyl chloride in Step 3 | HRMS (ESI) m/z [M+Na]+ calc'd for C24H18D3N5NaO 2: 437.1781, found: 437.1776 | +++ |
| **46** | 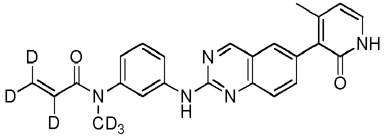 | Synthesized according to Synthesis Scheme IV, except using d3-iodomethane instead of iodoethane, and using d3-acryloyl chloride instead of acryloyl chloride in Step 3 | HRMS (ESI) m/z [M+H]⁺ calc'd for C24H16D6N5O2: 418.2150, found: 418.2142 | +++ |
| **47** | 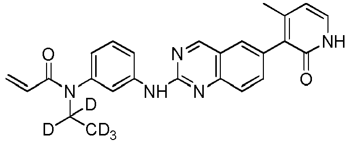 | Synthesized according to Synthesis Scheme IV, except using d5-iodoethane instead of iodoethane in Step 3 | HRMS (ESI) m/z [M+H]⁺ calc'd for C25H19D5N5O2: 431.2244, found: 431.2235 | +++ |
| **48** | 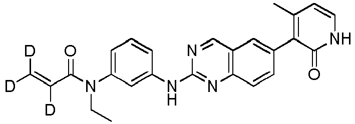 | Synthesized according to Synthesis Scheme IV, except using d3-acryloyl chloride instead of acryloyl chloride in Step 3 | HRMS (ESI) m/z [M+H]⁺ calc'd for C25H21D3N5O2: 429.2118, found: 429.2122 | +++ |
| **49** | 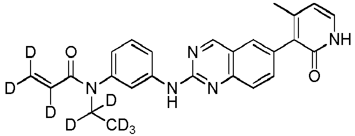 | Synthesized according to Synthesis Scheme IV, except using d5-iodoethane instead of iodoethane, and using d3-acryloyl chloride instead of acryloyl chloride in Step 3 | HRMS (ESI) m/z [M+H]⁺ calc'd for C25H16D8N5O2: 434.2432, found: 434.2430 | +++ |
| 50 | 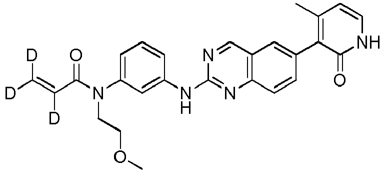 | Synthesized according to Synthesis Scheme IV, except using 2-methoxyl-1-iodoethane instead of iodoethane, and using d3-acryloyl chloride instead of acryloyl chloride | HRMS (ESI) m/z [M+H]⁺ calc'd for C26H23D3N5O3 : 459.2224, found: 459.2212 | +++ |

As can be seen from the efficacy data in the last column of Table 1 above, the compounds of the present application can achieve excellent inhibitory effects against Btk. In particular, the compounds of Examples 2, 5, 19, 23, 24, 26, 33, 34, 37, 38, 44, 45, 46, and 50 had particularly excellent inhibitory effects.

### Test Example 2: Covalent inhibition assay on BTK

This assay was carried out using the compounds of Examples 23 and 26. The reagents used are as follows:
BTK kinase: brand name: carna, product number: 08-180, concentration: 200 ng/µl;
DMSO: brand name: sigma, product number: D4540;
BTK enzyme activity buffer: 1× kinase buffer + 1 mM DTT + 5 mM MgCl₂ + 50 nM SEB;
BTK probe (100 µM): prepared according to Sci. Rep. 2015, 5, 16136 (Pan Zhengying, *et al*.);

The compounds of Examples 23 and 26: prepared as described above, and prepared at two concentrations (0.5 µM/5 µM) respectively.

The steps of the assay are as follows:
1. The sample order was 1% DMSO, 0.5 µM compound of Example 23, 5 µM compound of Example 23, 0.5 µM compound of Example 26, and 5 µM compound of Example 26. The compounds, BTK, probe and 2X loading buffer were added to 5 200 µl EP tubes using a pipette.
2. The final concentrations of the compounds of Examples 23 and 26 were 0.5 µM and 5 µM, and the control group was 1% DMSO. The compounds were first prepared with DMSO to 100X the above final concentrations, and diluted x40 with BTK enzyme activity Buffer. For the reaction, 4 µl/well of the compound was added to the 10 µl system using a pipette to reach its final concentration. For 1% DMSO, the stock solution was diluted x40 with BTK enzyme activity Buffer.
3. The BTK (200 ng/µl) was diluted to 10 ng/µl, and the probe (100 µM) was diluted to 2.5 µM. They were placed on ice for later use, and the probe was protected from light.
4. To each tube was added 4 µl of BTK, followed by 4 µl of the Example compound. After 1 h incubation, the probe was added, and then incubated for 1 h in the dark.
5. 2× loading buffer was prepared. After the probe was incubated, 10 µl of the 2× loading buffer was added to each well, mixed well, and heated at 100 °C for 10 min to prepare the sample.
6. 10 µl (containing 20 ng of BTK, and 0.5 µM probe) was loaded. Scanning was performed after SDS-PAGE. The order of samples was the order of spotting.

SDS-PAGE scanning results are shown in Fig. 1. Fluorescence scanning results showed that with 1% DMSO as a control, both compounds at a concentration of 0.5 µM could completely inhibit the labeling of BTK by the fluorescent probe, even at a concentration of 5 µM. If it is a compound that reversibly binds to BTK, the probe would bind to the released BTK during 1 h incubation with a mixture of the compound and BTK, leading to a fluorescent band. The fluorescence scanning results showed that after the compounds of Examples 23 and 26 were incubated with recombinant BTK for 1 h at the two concentrations, no free BTK could bind to the probe, which indicates that the compounds of Examples 23 and 26 irreversibly covalently binds to recombinant BTK.

### Test Example 3: Cell Viability assay

An *in vitro* cell viability assay was conducted using the compounds of Examples 1-50 following the steps below:
1. After OCI-LY7 cells were recovered, they were cultured at 37 °C, 5% CO₂, and 95% humidity.
2. For the compounds of Examples 1-50, 8 compound solutions at different concentrations were prepared, respectively. 50 µL of the compound solution at each concentration was added to a 96-well black plate.
3. The cell concentration was adjusted to about 30,000 cells/mL. 100 µL of cell suspension was added to the 96-well plate, and mixed well. The final cell density was about 3,000 cells/well.
4. The 96-well plate was placed at 37 °C, 5% CO₂, and 95% humidity for 72 h.
5. The cell survival rate was measured by the method of CellTiter-Glo® Luminescent Cell Viability Assay (Promega, Cat#G7572). The fluorescence data were read on a microplate reader (EnVision™, a multi-function microplate detector, PerkinElmer).
6. The obtained fluorescence data were analyzed using GraphPad Prism software. The cell survival rate was calculated for each example compound at each concentration, and the IC₅₀ interval of each example compound was obtained.

The IC₅₀ value of each example compound is listed in Table 2 below, where "+++" means IC₅₀ < 500 nM; "++" means 500 nM ≤ IC₅₀ < 2500 nM; and "+" means 2500 nM ≤IC₅₀.

**Table 2. Inhibition of each example compound on OCI-LY7 cells in vitro**

| Example | Results from inhibition of OCI-LY7 cells |
|---|---|
| 1 | +++ |
| 2 | +++ |
| 3 | +++ |
| 4 | +++ |
| 5 | +++ |
| 6 | +++ |
| 7 | +++ |
| 8 | +++ |
| 9 | ++ |
| 10 | +++ |
| 11 | +++ |
| 12 | +++ |
| 13 | +++ |
| 14 | +++ |
| 15 | +++ |
| 16 | ++ |
| 17 | ++ |
| 18 | +++ |
| 19 | +++ |
| 20 | ++ |
| 21 | +++ |
| 22 | +++ |
| 23 | +++ |
| 24 | +++ |
| 25 | +++ |
| 26 | +++ |
| 27 | +++ |
| 28 | +++ |
| 29 | ++ |
| 30 | + |
| 31 | +++ |
| 32 | ++ |
| 33 | +++ |
| 34 | ++ |
| 35 | +++ |
| 36 | +++ |
| 37 | +++ |
| 38 | +++ |
| 39 | ++ |
| 40 | ++ |
| 41 | +++ |
| 42 | ++ |
| 43 | + |
| 44 | +++ |
| 45 | +++ |
| 46 | +++ |
| 47 | +++ |
| 48 | +++ |
| 49 | +++ |
| 50 | +++ |

It is understood that the examples and embodiments described herein are for illustrative purposes only and that various modifications or changes in light thereof will be suggested to persons skilled in the art and are to be included within the spirit and purview of this application and scope of the appended claims. All publications, patents, and patent applications cited herein are hereby incorporated by reference in their entirety for all purposes.

## Claims

1. A compound of Formula (I): 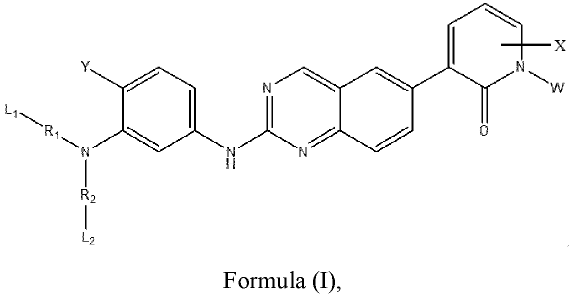 wherein,
W is selected from the group consisting of H, C₁₋₆alkyl, aryl optionally substituted with halogenated C₁₋₃alkyl or a five- to seven-membered nitrogen-containing aliphatic ring, heteroaryl, and -C(O)-R₃, wherein R₃ is C₁₋₃alkyl, di(C₁₋₃alkyl)amino, or C₁₋₃alkoxy optionally substituted with aryl;
X is selected from the group consisting of halo and C₁₋₆alkyl, and X is bonded to any bondable position on the pyridone ring;
Y is selected from the group consisting of H, halo, C₁₋₆alkyl, and C₁₋₃alkoxy optionally substituted with C₁₋₃alkoxy;
R₁ and R₂ are independently the same or different, and are selected from the group consisting of H, C₁₋₃alkyl optionally substituted with C₁₋₃alkoxy or di(C₁₋₃alkyl)amino, C(O), S(O), and S(O)₂;
L₁ and L₂ are independently the same or different, and are selected from the group consisting of C₁₋₆alkyl optionally substituted with -NR₄R₅, C₂₋₃alkenyl optionally substituted with halo or optionally substituted C₁₋₃alkyl, and C₂₋₃alkynyl optionally substituted with C₁₋₃alkyl, wherein the substituent of the optionally substituted C₁₋₃alkyl is di(C₁₋₃alkyl)amino, or a five- to seven-membered nitrogen-containing aliphatic ring; and
R₄ and R₅ in -NR₄R₅ are independently the same or different, and are selected from the group consisting of H, C₁₋₃alkyl, and C₂₋₃alkenylcarbonyl, or one of R₄ and R₅, and one carbon atom in the C₁₋₆alkyl optionally substituted with -NR₄R₅, together with the atom to which they are bonded, form a five- to seven-membered nitrogen-containing aliphatic ring,
provided that when R₁ is H, or C₁₋₃alkyl optionally substituted with C₁₋₃alkoxy or di(C₁₋₃alkyl)amino, L₁ is absent, and when R₂ is H, or C₁₋₃alkyl optionally substituted with C₁₋₃alkoxy or di(C₁₋₃alkyl)amino, L₂ is absent,
or a pharmaceutically acceptable salt thereof.

2. The compound or a pharmaceutically acceptable salt thereof according to claim 1, wherein the - N(R₁-L₁)(R₂-L₂) comprises at least one unsaturated carbon-carbon bond.

3. The compound or a pharmaceutically acceptable salt thereof according to claim 2, wherein the unsaturated carbon-carbon bond is a carbon-carbon double bond or a carbon-carbon triple bond.

4. The compound or a pharmaceutically acceptable salt thereof according to any one of claims 1-3, wherein,
W is selected from the group consisting of H, aryl optionally substituted with -CF₃ or 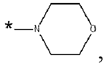 and -C(O)-R₃, wherein R₃ is C₁₋₃alkoxy optionally substituted with aryl;
Y is selected from the group consisting of H, halo, and C₁₋₃alkoxy;
R₁ and R₂ are independently the same or different, and are selected from the group consisting of H, C₁₋₃alkyl optionally substituted with C₁₋₃alkoxy or di(C₁₋₃alkyl)amino, and C(O);
L₁ and L₂ are independently the same or different, and are selected from the group consisting of C₁₋₆alkyl substituted with -NR₄R₅, C₂₋₃alkenyl optionally substituted with substituted C₁₋₃alkyl, and C₂₋₃alkynyl optionally substituted with C₁₋₃alkyl, wherein the substituent of the substituted C₁₋₃alkyl is dimethylamino, 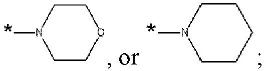 and
R₄ and R₅ in -NR₄R₅ are independently the same or different, and are selected from the group consisting of H, C₁₋₃alkyl, and C₂₋₃alkenylcarbonyl, or one of R₄ and R₅, and one carbon atom in the C₁₋₆alkyl substituted with -NR₄R₅, together with the atom to which they are bonded, form a tetrahydropyrrole ring.

5. The compound or a pharmaceutically acceptable salt thereof according to any one of claims 1-4, wherein,
W is selected from H;
X is selected from C₁₋₆alkyl;
Y is selected from the group consisting of H and C₁₋₃alkoxy;
R₁ and R₂ are independently the same or different, and are selected from the group consisting of H, C₁₋₃alkyl optionally substituted with C₁₋₃alkoxy, and C(O);
L₁ and L₂ are independently the same or different, and are selected from the group consisting of C₁₋₆alkyl substituted with -NR₄R₅, C₂₋₃alkenyl optionally substituted with di(C₁₋₃alkyl)amino C₁₋₃alkyl , and C₂₋₃alkynyl optionally substituted with C₁₋₃alkyl; and
R₄ and R₅ in -NR₄R₅ are independently the same or different, and are selected from the group consisting of H and C₂₋₃alkenylcarbonyl.

6. The compound or a pharmaceutically acceptable salt thereof according to any one of claims 1-5, wherein one of R₁ and R₂ is H or C₁₋₃alkyl optionally substituted with C₁₋₃alkoxy or di(C₁₋₃alkyl)amino, and the other is C(O).

7. The compound or a pharmaceutically acceptable salt thereof according to any one of claims 1-6, wherein one of Ri and R₂ is H, and the other is C₁₋₃alkyl optionally substituted with C₁₋₃alkoxy or di(C₁₋₃alkyl)amino.

8. The compound or a pharmaceutically acceptable salt thereof according to any one of claims 1-7, wherein the -N(R₁-L₁)(R₂-L₂) is selected from the group consisting of -NR₆-C(O)-CH=CH₂, -NR₆-C(O)-C≡C-CH₃, -NR₆-C(O)-CH=CH₂CH₂N(CH₂)₂, 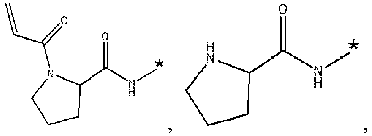 and 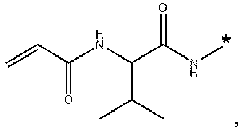 where R₆ is H, or C₁₋₃alkyl optionally substituted with C₁₋₃alkoxy.

9. The compound or a pharmaceutically acceptable salt thereof according to any one of claims 1-8, wherein the -N(R₁-L₁)(R₂-L₂) is selected from the group consisting of

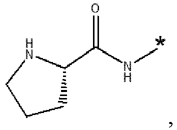

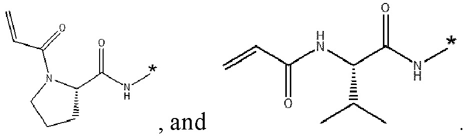

10. The compound or a pharmaceutically acceptable salt thereof according to any one of claims 1-9, wherein W is selected from H, X is selected from methyl, Y is selected from the group consisting of H and methoxy, and the -N(R₁-L₁)(R₂-L₂) is -NR₆-C(O)-CH=CH₂, wherein R₆ is H, or C₁₋₃alkyl optionally substituted with C₁₋₃alkoxy.

11. The compound or a pharmaceutically acceptable salt thereof according to any one of claims 1-10, wherein,
when Y is C₁₋₆alkyl, or C₁₋₃alkoxy optionally substituted with C₁₋₃alkoxy, and one of R₁ and R₂ is C₁₋₃alkyl optionally substituted with C₁₋₃alkoxy or di(C₁₋₃alkyl)amino, one of R₁ and R₂, and Y, together with the atom to which they are bonded, can form a five- to seven-membered nitrogen-containing aliphatic ring.

12. The compound or a pharmaceutically acceptable salt thereof according to any one of claims 1-11, wherein when Y is C₁₋₃alkoxy optionally substituted with C₁₋₃alkoxy, and one of R₁ and R₂ is C₁₋₃alkyl optionally substituted with C₁₋₃alkoxy or di(C₁₋₃alkyl)amino, one of R₁ and R₂, and Y, together with the atom to which they are bonded, can form a morpholine ring.

13. The compound or a pharmaceutically acceptable salt thereof according to any one of claims 1-12, wherein the compound is selected from the group consisting of: 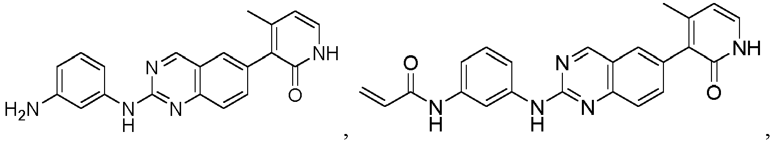 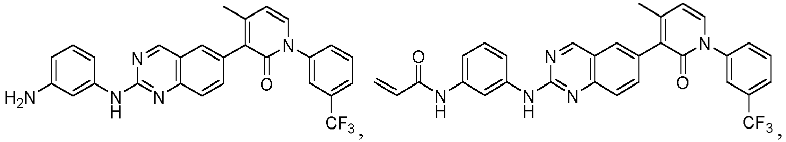 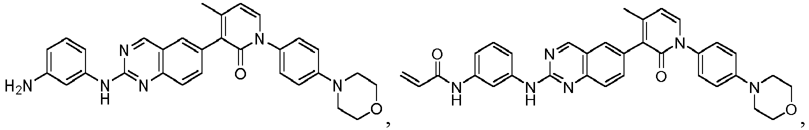 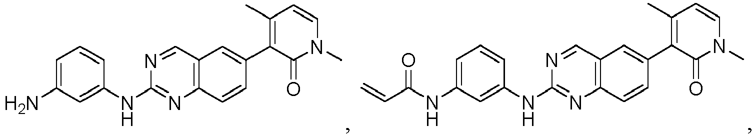 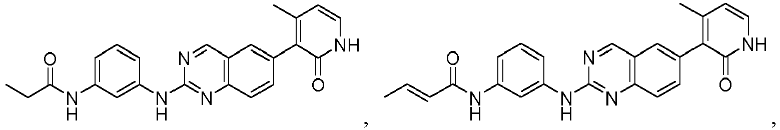 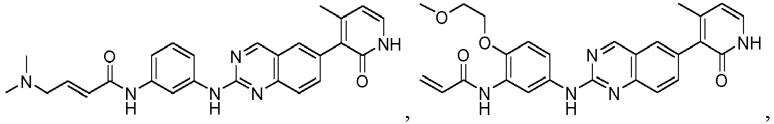 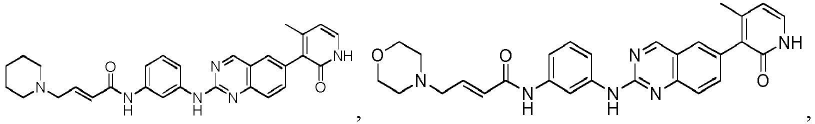 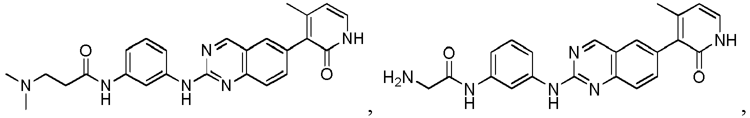 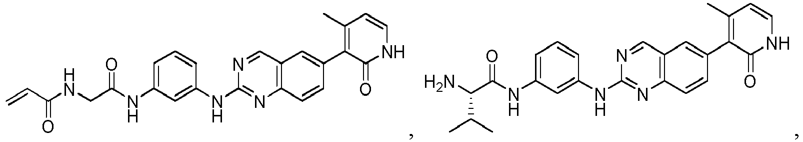 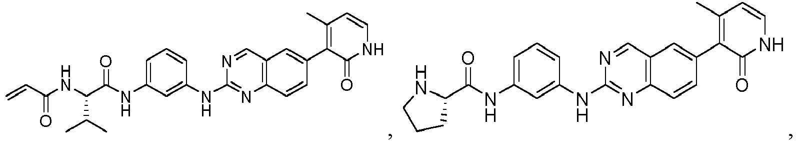 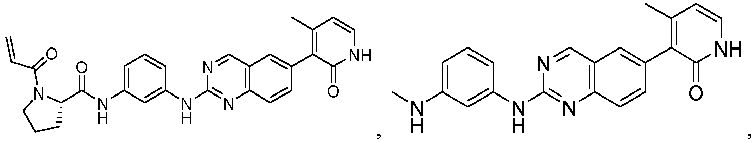 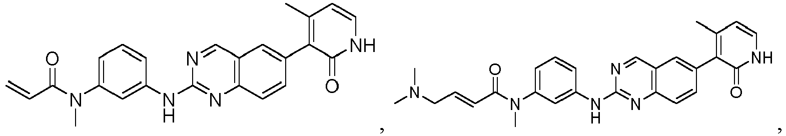 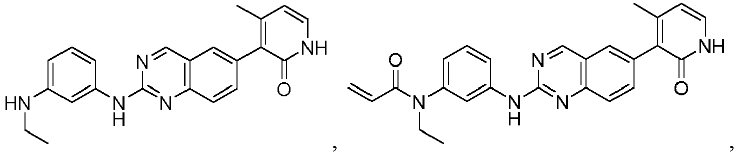 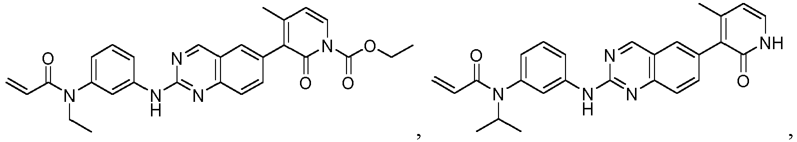 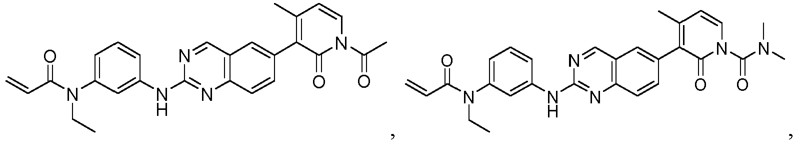 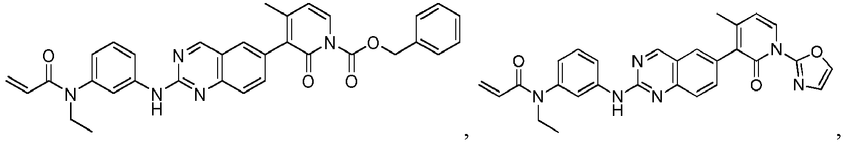 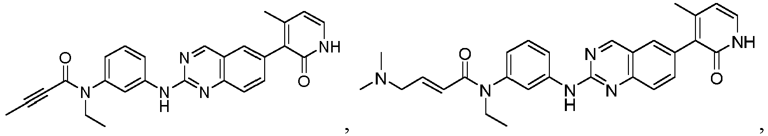 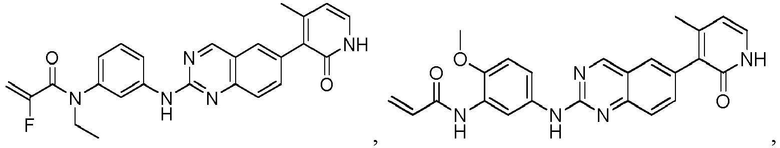 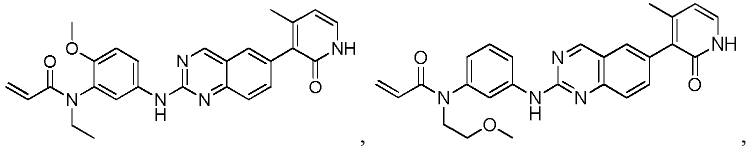 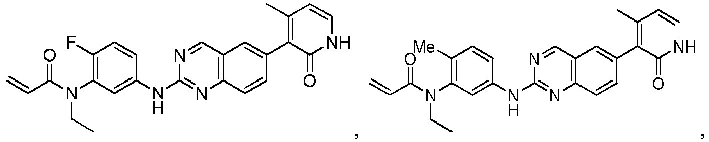 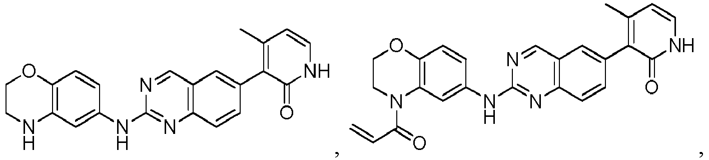 and

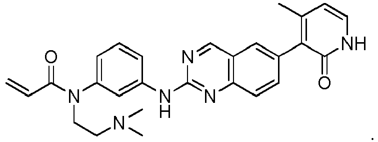

14. The compound or a pharmaceutically acceptable salt thereof according to any one of claims 1-13, wherein the compound is further selected from the group consisting of:

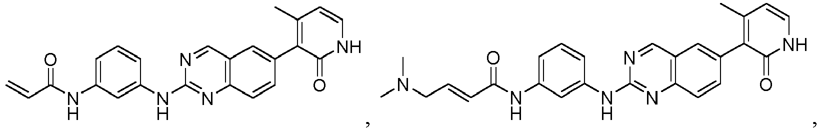

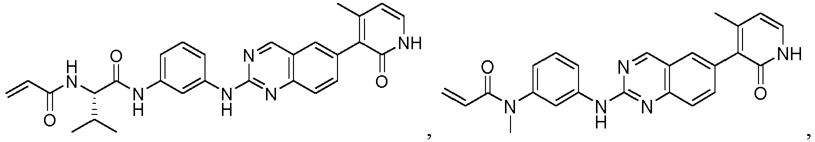

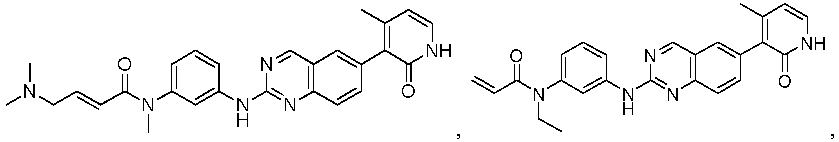

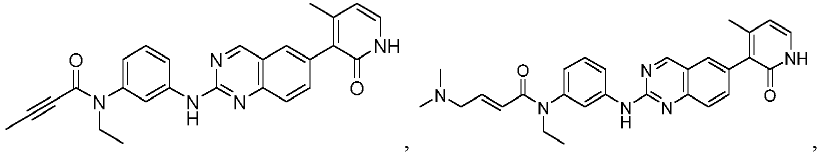

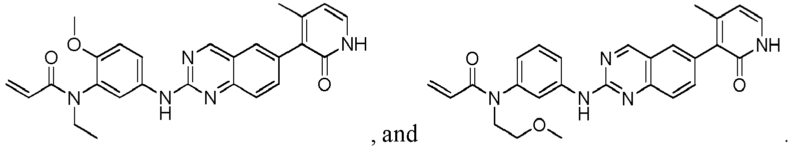

15. The compound or a pharmaceutically acceptable salt thereof according to any one of claims 1-14, wherein the salt is an acid addition salt.

16. The compound or a pharmaceutically acceptable salt thereof according to claim 15, wherein the acid addition salt is an inorganic acid addition salt or an organic acid addition salt.

17. The compound or a pharmaceutically acceptable salt thereof according to any one of claims 1-14, wherein the salt is a salt formed by replacing an acidic proton in the compound with a metal ion, or a salt formed by coordination of the compound with an organic base or inorganic base.

18. A pharmaceutical composition comprising a therapeutically effective amount of the compound or a pharmaceutically acceptable salt thereof according to any one of claims 1-17, and a pharmaceutically acceptable carrier or excipient.

19. Use of the compound or a pharmaceutically acceptable salt thereof according to any one of claims 1-17 in the manufacture of a medicament for inhibiting the activity of Bruton's tyrosine kinase.

20. The use according to claim 19, wherein the inhibiting is performed by covalent binding.

21. The use according to claim 20, wherein the inhibiting is achieved by forming a covalent bond between the compound and an amino acid residue on Bruton's tyrosine kinase.

22. The use according to any one of claims 19-21, wherein the medicament is used to treat diseases selected from the group consisting of autoimmune diseases, xenogeneic immune diseases, inflammatory diseases, cancers, and thromboembolic diseases.
